# EUROPEAN PATENT SPECIFICATION

(11) **EP 2 958 607 B1**
(45) Date of publication and mention of the grant of the patent: **22.06.2016**
(21) Application number: 14720973.8
(22) Date of filing: 01.05.2014
(51) Int. Cl.: A61L 29/08, A61L 29/16

(54) **BALLOON SURFACE COATING**
BALLONOBERFLÄCHENBESCHICHTUNG
REVÊTEMENT DE SURFACE DE BALLONNET

(30) Priority: 02.05.2013 WO PCT/EP2013/059191; 01.10.2013 WO PCT/EP2013/002936
(43) Date of publication of application: 30.12.2015
(73) Proprietor: Cardionovum GmbH, 53227 Bonn (DE)
(72) Inventor: ORLOWSKI, Michael, 53173 Bonn (DE)
(74) Representative: Arth, Hans-Lothar
(86) International application number: PCT/EP2014/058959
(87) International publication number: WO 2014/177678

(56) References cited:
- EP-A1- 2 243 501
- WO-A1-2005/020708
- WO-A1-2009/064429
- WO-A1-2013/007273
- WO-A2-2008/046641
- STUMMER S ET AL: "Application of shellac for the development of probiotic formulations", FOOD RESEARCH INTERNATIONAL, ELSEVIER APPLIED SCIENCE, BARKING, GB, vol. 43, no. 5, 1 June 2010 (2010-06-01), pages 1312-1320, XP027099814, ISSN: 0963-9969 [retrieved on 2010-06-01]
- Zuhair Osman: "Investigation of Different Shellac Grades and Improvement of Release From Air suspension Coated Pellets", , 1 January 2012 (2012-01-01), pages 1-115, XP055120845, Retrieved from the Internet: URL:http://ubm.opus.hbz-nrw.de/volltexte/2 013/3325/pdf/doc.pdf [retrieved on 2014-05-30]

## Description

The present invention relates to balloon catheter coated with an active agent and a shellac alkali salt or preferably a shellac ammonium salt. Moreover the present invention relates to a method for coating catheter balloons with a pharmacological active agent and an aqueous solution of shellac.

Implantation of vessel grafts such as stents has become a well-established surgical intervention for the treatment of stenosis. In this context, so-called restenosis (recurrent stenosis), i.e. the reocclusion of the vessel is a frequently occurring complication. There's no exact definition of the term restenosis to be found in literature. The most frequently used morphological definition of restenosis defines restenosis as a reduction of the vessel diameter to less than 50% of the normal value subsequent to successful PTA (percutaneous transluminal angioplasty). Said definition describes an empirically determined value and its hemodynamic meaning and association with clinical symptoms lack scientific background. In practice, clinical deterioration in a patient is often considered a sign for the occurrence of restenosis in the previously treated vessel section.

To avoid such problems, a so-called "biological stenting" may be performed using only a coated catheter balloon without any stent, i.e. the vessels are dilated at a constricted site by the dilatation of a coated catheter balloon, wherein, while the catheter balloon is dilated for a short period of time, a sufficient amount of pharmacological agent is transferred to the vessel wall to avoid re-constriction or reocclusion of the vessel due to the dilatation of the vessel and the delivery of active agents.

Nowadays, it is known that active agents can be applied to a balloon catheter with various matrix-substances, including substances such as the terpenoid shellolic acid. The active agents are released during the balloon inflation at the stenosis, in order to penetrate the arterial wall segment, in order to evolve their antiproliferative and anti-inflammatory effects on the smooth muscle cells and to suppress proliferation in the vessel lumen.

Suppression of cellular reactions is mainly accomplished during the first days and weeks by means of preferably antiproliferative, immunosuppressive and/or antiphlogistic agents and their likewise active derivatives /analogues and metabolites.

The international patent application WO 2004/028582 A1 discloses multifold balloons which are coated, especially within the folds, with a composition of a pharmacological agent and a contrast medium. A method for spray coating catheter balloons is described in WO 2004/006976 A1.

WO 2008/046641 discloses coatings for implants, not mentioning catheter or catheter balloons comprising a combination of shellac and paclitaxel. Thereby WO 2008/046641 refers to stents in particular showing in vitro release kinetics of stents coated with 1.0% / 0.5% shellac composite. It was shown that stents coated with rapamycin only released the drug more efficiently in contrast to shellac and rapamycin coated stents, which released the drug much slower. Shellac was deemed to be useful to modulate the release kinetics of an implant-based, e.g. stent-based compound to slow the release kinetic (more than 60 days). Such a retardation of drug release is not favourable for a catheter balloon, where it is the main goal, in contrast to a stent, to release as much of the coated drug in a time frame as short as possible.

EP2421572 discloses coating method of a catheter balloon using a solution of paclitaxel together with shellac in a suitable organic solvent such as acetone, ethyl acetate, ethanol, methanol, DMSO, THF, chloroform, methylene chloride.

WO2013/007273 discloses catheter balloons coated with a gradient coating comprising an active agent and shellac. The authors of a publication in Circulation 2004, Vol. 110, 810 - 814 demonstrated that catheter balloons coated with pure Paclitaxel did not show any therapeutic effect. A therapeutic effect was only achieved when the Paclitaxel was combined with the contrast agent solution ULTRAVIST^{®} ULTRAVIST^{®} is a solution of the contrast agent iopromide. The same observation was made by Cremers et al., Clin. Res. Cardiol., 2008, 97 - Suppl.1.

Therefore, it is an objective of the present invention to apply an active agent, especially preferred the active agent paclitaxel or sirolimus, onto a catheter balloon in such a manner that a coating is created which is homogenously detached from the balloon and can be effectively transferred to the vessel wall so that an optimal bioavailability of the active agent and a therapeutic effect concerning reduction of restenosis can be achieved.

Said objective is solved by the technical teaching of the independent claims. Further advantageous embodiments of the invention result from the dependent claims, the description, the figures and the examples.

Surprisingly it has been found that a catheter balloon comprising a coating with an active agent and a shellac alkali salt or preferred a shellac ammonium salt, is suited for resolving said objective.

Thus the present invention relates to a catheter balloon comprising a coating with an active agent and a water soluble shellac salt such as a shellac alkali salt or a shellac ammonium salt. In the following such a coating with an active agent and a water soluble shellac salt such as a shellac alkali salt, preferred a shellac ammonium salt, is called "Shellaqua"-coating. The preferred water soluble salt of shellac is a shellac ammonium salt. The term "water soluble" refers to a solubility in water at 25°C of at least 30 g/L, preferably of at least 40 g/L, preferably of at least 50 g/L, preferably of at least 60 g/L, preferably of at least 70 g/L, preferably of at least 80 g/L, preferably of at least 90 g/L, and most preferably at 25°C of at least 100 g/L. Thus the term "Shellaqua" refers to a coating comprising or consisting of a water soluble shellac salt, especially an ammonium salt of shellac, and an active agent, preferably an antirestenotic agent and most preferably paclitaxel or rapamycin. This coating called Shellaqua may further comprise a fatty acid, preferably an unsaturated fatty acid but does preferably not contain any further ingredients and does especially not contain synthetic polymers. Thus the Shellaqua coating preferably consists of a water soluble shellac salt, especially an ammonium salt of shellac and an antirestenotic agent preferably paclitaxel or rapamycin or consists of a water soluble shellac salt, especially an ammonium salt of shellac and a fatty acid preferably an unsaturated fatty acid and an antirestenotic agent preferably paclitaxel or rapamycin. The term "ammonium" refers to NH₄⁺.

The inventor could show that using a catheter balloon having the inventive "Shellaqua"-coating increases the amount of active agent transferred during balloon inflation about ten times compared to a catheter balloon coated with the active agent and shellac in its acid form. Furthermore it's the first time that such high concentrations of the active agent paclitaxel could be observed in the vessel wall after deployment of a coated catheter balloon. The "Shellaqua"-coating is apparently much better suited to facility a transfer of the active agent from the catheter balloon to the vessel wall than shellac in its acidic form.

The term "alkali salts" or "alkali" as used herein refer to a basic, ionic salt of an alkali metal or alkaline earth metal element. The water soluble shellac salt also called herein shellac alkali salt may be a potassium salt, an ammonium salt, a basic amino acid salt and/or a mixture thereof.

Shellac is the general term for the refined form of lac, a natural polyester resin secreted by insects. Lac insects belong to the order of Hemiptera, superfamily Coccoidea such as Metatachardia, Laccifer, Tachordiella, and others, however, members of two families - Lacciferidae and Tachardinidae are more prominent in lac secretion. The one that is commercially cultured is Kerria lacca, which is also known by such synonyms as Laccifer lacca Ker, Tachardia lacca, and Carteria lacca. Kerria lacca is an Indian scale insect, which infests branches of numerous trees from the East Indies, such as Butea frondos Rosch, Acacia arabica Willd and Ficus religiosa Linn. Broken branches are sold as stick lac and, after grounding and washing with water to eliminate wood and red pigments (lac dye), seed lac is obtained. Raw material shellac consists of 70-80% resin, 4-8% dye, 6-7% hard and high gloss finished wax, 3% water, up to 9% vegetable and animal impurities and aroma substances. Purification of seed lac gives the more homogeneous product known as shellac. The major components of shellac are aleuritic, jalaric and shellolic acids, as well as butolic and kerrolic acids. Seed lac and orange shellac contain approximately 5-6% wax and two coloring components, the water soluble laccaic acid and the water insoluble erythrolaccin.

A possibility for chemical description of resin molecule is a structure model where in each case 4 molecules jalaric or laccijalaric acid and aleuritic acid are connected by ester bonding alternately.

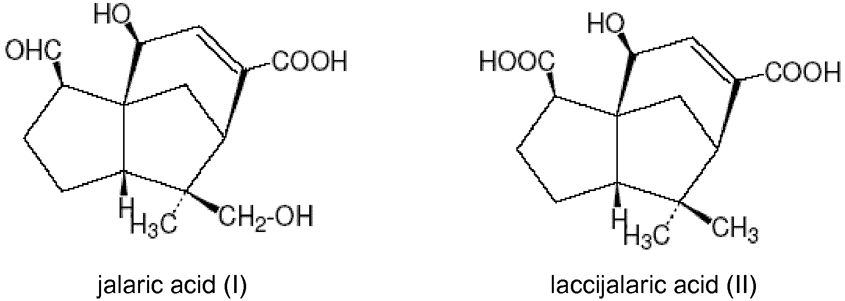

Its chemical composition is almost constant, although the amount of some components changes depending on the nature of host trees on which the insects grows. By Cannizzaro-type disproportionation under alkaline hydrolysis will be synthesized from these acids shellolic acid (IV) and derivate compounds. Purified shellac consists of two main components. These components are 9,10,16-trihydroxypalmitic acid (aleuritic acid) CAS [53-387-9] and shellolic acid (IV).

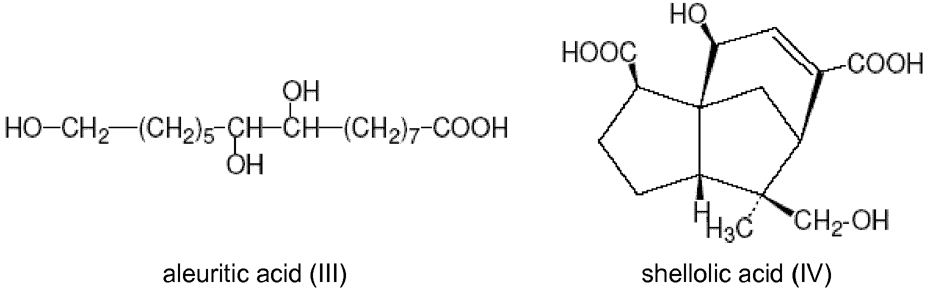

Under the general name shellac, many types or grades of shellac are commercially available. Their properties and color depend on the raw material (seedlac), the method for refining, and the processing parameters. Three very different processes are used for refining the seed lac to shellac (bleaching, melting, and solvent extraction), resulting in products with different characteristics and properties.

By the bleaching process refined bleached or white shellac is obtained by dissolving seed lac in an aqueous alkaline solution, which is then filtered, dewaxed, and bleached with sodium hypochlorite to completely remove the color. However, changes in the molecular structure and the addition of chlorine substituents may lead to self-crosslinking and polymerization.

After melting the seed lac, the highly viscous molten lac is pressed through a filter and drawn to a thin film. Once cooled, the film breaks into thin flakes. The shellac wax is not removed by this process and the color depends on the type of seed lac used.

Solvent extraction is a very gentle process for refining shellac. The seed lac is dissolved in ethanol, and wax and impurities are removed by filtration. Activated carbon is used to produce light-colored grades. After a further filtration step and the removal of ethanol, the resin is drawn to a thin film, which breaks into flakes after cooling. The properties of the final product depend on the type of seed lac used and are influenced by the processing parameters and the grade of activated carbon.

Followings are the commercial grades of shellac:
- Seedlac
- Hand Made Shellac
- Machine Made Shellac
- PhEur 7 European Pharmacopoeia Edition 7 specifies: Bleached shellac, Bleached Dewaxed Shellac, Wax-containing Shellac, and Dewaxed Shellac
- The United States Pharmacopeia and The National Formulary (USP-NF) specifies. Regular bleached shellac, Refined bleached shellac, Orange shellac, and Dewaxed orange shellac.

Shellac is widely used as a moisture barrier coating for tablets and pellets due to its low water vapor and oxygen permeability. Shellac has been used for pharmaceutical and controlled release coatings for a long time. It has usually been applied in the form of alcoholic solutions (pharmaceutical glazes) or solutions using other organic solvents.

Shellac, like other polymers with carboxyl groups, is not soluble in water. It is soluble in ethanol, methanol and partially soluble in ether, ethyl acetate and chloroform. However, it is possible to prepare aqueous shellac solutions of alkali salts or ammonium salts. The selection of the base and the method for dissolving will influence the properties of the film, in regard to the present invention ammonium is preferable. Therefore ammonium carbonate was chosen as a preferred base. Another preferred embodiment refers to ammonium bicarbonate as base. Ammonium bicarbonate is also known as ammonium hydrogen carbonate (NH₄HCO₃). The preferred water soluble shellac salt in regard to the present invention is shellac ammonium salt having CAS number [68308-35-0].

There are several disadvantages associated with the use of organic solvents in general:
1. They are flammable and toxic
2. Their vapor causes hazards to the coating equipment operator
3. High cost of solvent
4. Solvent residue in formulation

Alcoholic solutions of shellac or in general shellac solution in an organic solvent have the disadvantage that during the process of coating a certain degree of active agent evaporates too, which makes it more difficult to ensure a uniform amount of active agent in the coating. Furthermore the repeatability is worse.

It has been found that aqueous alkali shellac solutions, preferably ammonium shellac solutions, based on dewaxed orange shellac, do not show problems exhibited by alcoholic shellac solutions and have very stable release characteristics even after extended storage times. Furthermore, they can be formulated in combination with other water soluble polymers such as HPMC, CMC, alginates, or modified starch eventually together with plasticizers.

The invention is also directed to coating methods of the following type which are especially suited for manufacturing a balloon catheter with a coated balloon according to the present invention.

One method of the invention for loading or coating balloon catheter comprises the following steps:
I) providing an uncoated balloon catheter;
   and
IIA) providing an aqueous solution of an active agent and shellac;
   or
IIB) providing a solution of an active agent and providing an aqueous solution of shellac;
   and
IIIA) coating the surface of the balloon of the balloon catheter with the aqueous solution of the active agent and shellac;
   or
IIIB) coating the surface of the balloon of the balloon catheter with the solution of the active agent and subsequently with the aqueous solution of shellac or coating the surface of the balloon of the balloon catheter with the aqueous solution of shellac and subsequently with the solution of the active agent;
IV) drying the coated catheter balloon.

Thereby it is preferred that the aqueous solution of shellac or the aqueous solution of the active agent and shellac are prepared using a solution of an alkali salt, more preferably an ammonium salt. The term "uncoated" as used herein refers to a catheter balloon with a smooth or structured or roughened surface without any drug coating, i.e. the balloon surface does not comprise a pharmaceutically active agent and especially no anti-proliferative, anti-angiogenic or anti-restenosis drug and no coating containing an anti-proliferative, anti-angiogenic or anti-restenosis drug. Of course the coating steps IIIA) and IIIB) respectively, may be repeated several times, with or without a drying step in between.

It is further preferred that said method comprises further a step D' after step D):
D') applying the aqueous solution of shellac again

Of course drying steps can follow after each coating step so a more detailed method reads as follows:
A) providing an uncoated balloon catheter;
   and
B) providing an solution of an active agent and providing an aqueous solution of shellac;
   and
C) coating the surface of the balloon of the catheter balloon with the aqueous solution of shellac and drying the coated balloon surface;
   and
D) applying the solution of the active agent and drying the coated balloon surface
   and subsequently
E) drying the coated catheter balloon.

Thereby it is preferred that the solution of an active agent is an aqueous solution, too. The application of aqueous shellac solutions of water soluble shellac salts, such as Aqualacca^{®} or Aquagold^{®} does not only avoid the problems with organic solvent systems but also reproves the performance of the obtained coating by stable dissolution or respectively release characteristics, especially after extended storage time and result in improved mechanical properties compared to coatings comprising shellac but not the basic shellac salt. A balloon catheter coated using a shellac salt according to the invention and especially an ammonium shellac salt has a coating being less brittle so that less particles of the coating crumble away during deployment. Less number or particles released during catheter balloon placement decrease the risk of micro embolism clearly. The solubility and transfer rate of the coating is increase by using a basic shellac salt instead of shellac as such. It seems that this increase in solubility is caused by the presence of shellac as basic salt not by the solvent. Therefore it is also possible to use an aqueous solution of an ammonium salt of shellac sediment the salt and the active agent and solve the resulting pellet in an organic solvent in order to coat the catheter balloon. This method is preferred if the used active agent is not soluble in an aqueous solution.

One aspect of the method according to the invention comprises that a catheter balloon and preferably an uncoated catheter balloon or a catheter balloon without any releasable active agent in its surface is provided. Than a solution of an active agent and an aqueous shellac solution is prepared and applied sequentially using conventional coating methods such as spray coating, dip coating etc. in order to obtain after the drying step a solid coating on the surface of the catheter balloon.

Another aspect of the inventive method comprises that one aqueous solution containing an active agent and shellac is prepared. Subsequently, this solution is applied on the surface of a catheter balloon and preferably an uncoated catheter balloon or a catheter balloon without any releasable active agent in its surface using conventional coating methods like the one mentioned above. Shellac contains carboxyl groups. It is not soluble in water, but it can dissolve at higher pH, so it is possible to prepare aqueous shellac solutions of alkali salts or ammonium salts. Therefore the term "aqueous solution of shellac" as used herein refers always to shellac dissolved in aqueous solution of inorganic alkalis so that a shellac alkali salt originates. By the physical drying of the aqueous solution of shellac a film of the shellac alkali salt forms wherein at least one active agent is incorporated. The term "inorganic alkali" refers to substances which are basic in water (pH > 7.0) and which contain cations which form a water soluble salt with shellac.

Aqueous solutions are easy to handle and allow the production of films that lack the aging instability of films made using organic solvents. Therefore using aqueous shellac solutions the performance of the resulting polymer film is improved by stable dissolution characteristics even after extended storage time.

A suitable alkali salt in regard to this invention may be selected from the group consisting of sodium bicarbonate, sodium carbonate, calcium hydroxide, calcium bicarbonate and calcium carbonate, potassium bicarbonate, potassium carbonate, ammonia, ammonium carbonate, and ammonium bicarbonate. Preferably the salt is a solution of an alkali salt is a solution of ammonia, ammonium carbonate, or ammonium bicarbonate. The solutions may be prepared by dissolved shellac directly in the alkali solution. For example shellac is directly dissolved in ammonium carbonate solution and the excess ammonia evaporates as NH₃. Alternatively a ready to use aqueous shellac solution may be used like AQUALACCA 25^{®} distributed by Chemacon GmbH or SSB AQUAGOLD (based on shellac SSB 57 dewaxed Orange shellac) distributed by Stroever GmbH & Co. KG. It is preferred that the aqueous solution of shellac alkali salt, preferably shellac ammonium salt, contains 10 - 30% solids, more preferably 20 - 25% solids and has a pH of 7 - 7.5. The viscosity of the coating solution containing the shellac alkali salt according to DIN cup 4mm is preferably < 25 sec.

In one coating method according to the present invention step D is carried out in a way that the solution of the active agent penetrates the layer of shellac alkali salt. Thereby a concentration gradient originates. Preferably the layer of shellac alkali salt should not soak the solution of the active agent till the surface of the catheter balloon. This means directly on the surface of the catheter balloon stays a base coat or a zone in the layer of shellac alkali salt which is free of the active agent. Hence, preferably the catheter balloon has a base coat which consists of shellac alkali salt only. The concentration of the active agent preferably increases from zero, or nearly zero, to the maximum with increasing distance from the balloon surface. Within the coating of the catheter balloon there could be one zone or layer consisting of pure active agent on top of the coating.

The drying step E) or IV) can be performed at room temperature or at elevated temperatures up to 50°C and at atmospheric pressure or under reduced pressure to high vacuum. The drying step is also possible after the surface of the catheter balloon has been coated firstly with the aqueous solution of shellac and after the layer of the active agent has been applied. Thereby the first drying steps are preferably conducted at room temperature and atmospheric pressure, while preferably after the last coating step of the method the drying step is more intensive, i.e. longer or with vacuum or with elevated temperature.
One preferred method of the invention for loading or coating dilatable catheter balloons comprises the following steps:
IA) providing an uncoated balloon catheter;
   and
IIA) providing an aqueous solution of an active agent and shellac;
   and
IIIA) coating the surface of the catheter balloon with the aqueous solution of the active agent and shellac;
   and
IV) drying the coated catheter balloon,
wherein the aqueous solution of shellac is prepared using a solution of an alkali salt, more preferably an ammonium salt.

Another aspect of the present invention is directed to a method for coating a balloon catheter according to claim 1 comprising the following steps:
IA) providing an uncoated balloon catheter;
   and
IIA) providing an aqueous solution of an active agent and a water soluble shellac salt;
   or
IIB) providing a solution of the active agent and providing an aqueous solution of a water soluble shellac salt;
   and
IIIA) coating the surface of the balloon of the balloon catheter with the aqueous solution of the active agent and the water soluble shellac salt;
   or
IIIB) coating the surface of the balloon of the balloon catheter with the solution of the active agent and subsequently with the aqueous solution of the water soluble shellac salt or coating the surface of the balloon of the balloon catheter with the aqueous solution of the water soluble shellac salt and subsequently with the solution of active agent;
IV) drying the coated balloon,
wherein the aqueous solution of the water soluble shellac salt or the aqueous solution of the active agent and the water soluble shellac salt are prepared using an alkali salt or an ammonium salt of shellac.

The solution or aqueous solution of the ammonium salt of shellac is preferably a solution of ammonia, ammonium carbonate, or ammonium bicarbonate and shellac.

The present invention includes further a method for loading or coating dilatable catheter balloons comprising the following steps:
IA) providing an uncoated catheter balloon; and
IIB) providing a solution of an active agent and an aqueous solution of shellac;
   and
IIIB) coating the surface of the catheter balloon with the solution of the active agent and subsequently with the aqueous solution of shellac or coating the surface of the catheter balloon with the aqueous solution of shellac and subsequently with the solution of the active agent;
IV) drying the coated catheter balloon.
wherein the aqueous solution of the active agent and shellac is prepared using a solution of an alkali salt, more preferably an ammonium salt.

An inventive coating method can optionally further comprise step V):
V) Sterilization of the active agent and shellac alkali salt coated catheter balloons.
The sterilization is most preferably performed with ethylene oxide.

The invention is furthermore directed to a catheter balloon comprising a coating with an active agent and shellac alkali salt and optionally a base coat, and/or a top coat. The term "base coat" as used herein refers to a layer of the coating of a catheter balloon which is immediately on the surface of the catheter balloon. This layer is a first layer which directly overlays the material of the catheter balloon. The term "top layer" or "topcoat" as used herein refers to a layer of the coating free of an active agent which overlays the active agent containing layer.

Another embodiment of the present invention relates to a catheter balloon comprising a "Shellaqua"-coating, wherein the coating comprises a concentration gradient of the active agent. Thereby the concentration gradient of the active agent is in the layer of shellac alkali salt as a matrix substance. This concentration gradient is referred herein as radial or vertical concentration gradient, because the concentration of the active agent increases from the surface of the balloon to the top or the surface of the coating or in other words the concentration of the active agent decreases from the top of the coating where the concentration is preferably between 90% by weight to 100% by weight to the surface of the catheter balloon where the concentration of the active agent is preferably between 0% by weight and 10% be weight.

In addition to this vertical concentration gradient a longitudinal or horizontal concentration gradient can be present so that the concentration of the active agent decreases from the middle of the catheter balloon to the distal end and proximal end of the catheter balloon. Thus the term "vertical concentration gradient" or "radial concentration gradient" as used herein refers to a decreasing concentration of the active agent and especially of paclitaxel from the top of the coating in direction to the balloon surface.

The term "gradient" as used herein refers to is a concentration gradient. This means that in the "Shellaqua"-coating of the catheter balloon according to the invention is a gradual difference in the concentration of the active agent, preferably of paclitaxel or sirolimus, in the matrix of shellac alkali salt between two regions. It is preferred that this regions are located radial or vertical to the catheter balloon that the lowest concentration of the active agent, like paclitaxel or sirolimus is directly on the surface of the catheter balloon (on the base material the balloon is made of) and the highest concentration is on top of the coating, which means on the end which comes in contact to the tissue. Exceptions are the embodiments which comprise a top coat of pure active agent. There it is preferred that the highest concentration is on top of the active agent containing layer, which means directly below the top coat. Further preferred is that the catheter balloon of the invention has more than one gradient, which means that there are gradual differences in the concentration of the active agent, preferably of paclitaxel, in shellac between four regions. Thereby the direction of said gradients should differ. It is especially preferred that beside the radial gradient described a longitudinal or horizontal gradient is present in the balloon coating, which means that the longitudinal or horizontal is an additional concentration gradient to the radial gradient. Here the regions are located longitudinal to the catheter balloon, so that for example the lowest concentration of the active agent, like paclitaxel is directly at one or both ends of the catheter balloon (where the balloon ends and the catheter or the catheter tip starts) and the highest concentration is in the middle of the balloon.
The term "longitudinal concentration gradient" or "horizontal concentration gradient" as used herein refers to a decreasing concentration of the active agent and especially of paclitaxel from the middle or middle part of the balloon surface to the proximal end as well as the distal end of the catheter balloon.

Preferably the coating of the catheter balloon comprises further a base coat of shellac as a first layer under the active agent layer. Also preferred is a catheter balloon, wherein the coating comprises further a top coat of shellac or of a polyether, especially of polyethylene glycol (PEG). The top coat of a polyether is especially preferred if the active agent in the balloon coating is sirolimus.

Preferred is a catheter balloon, wherein the active agent is an antiproliferative, immunosuppressive, anti-angiogenic, anti-inflammatory, and/or anti-thrombotic agent which are herein just called antirestenotic agent. It is preferred if the active agent or the antirestenotic agent is selected from the group consisting of or comprising:
abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-AII, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids and derivatives thereof, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1 a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, β-lapachone, podophyllotoxin, podophyllic acid-2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, S-nitroso derivatives, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel and derivatives thereof, 6-α-hydroxy-paclitaxel, taxoteres, paclitaxel bound to albumin, like nap-paclitaxel, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, isoiridogermanal, maytenfoliol, effusantin A, excisanin A and B, longikaurin B, sculponeatin C, kamebaunin, leukamenin A and B, 13,18-dehydro-6-alphasenecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), rapamycin derivatives, biolimus A9, pimecrolimus, everolimus, zotarolimus, tacrolimus, sirolimus bound to albumin, like nap-sirolimus fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

Basically any active agent as well as combination of active agents can be used, wherein, however, paclitaxel and paclitaxel derivatives, taxanes, docetaxel, paclitaxel bound to albumin, like nap-paclitaxel, as well as sirolimus and rapamycin derivatives as e.g. biolimus A9, pimecrolimus, everolimus, zotarolimus, tacrolimus, sirolimus bound to albumin, like nap-sirolimus fasudil and epothilones are preferred and particularly preferred are paclitaxel and sirolimus. The use of sirolimus is preferred since in contrast to paclitaxel, siromlimus, a hydrophilic macrolid antibiotic, is highly water soluble. Especially preferred are paclitaxel and rapamycin (i.e. sirolimus). Thus all ranges and values given herein and all embodiments disclosed herein are especially in regard to paclitaxel or sirolimus and should be first of all interpreted in this way.

Therefore the present invention relates to a balloon catheter comprising a "Shellaqua"-coating with paclitaxel as active agent. Another embodiment of the present invention relates to a balloon catheter comprising a "Shellaqua"-coating with sirolimus.

It was surprisingly found that an "Shellaqua"-coating, comprising paclitaxel or sirolimus is therapeutically highly useful in keeping blood vessels open, in reducing the late lumen loss and in reducing restenosis. The film which results from the aqueous shellac solution after drying is more elastic or less friable compared to the coatings obtained with alcoholic solutions so that an optimized transfer of the active agent to the lesion site is obtained. Furthermore this causes that the risk of thrombosis is reduced.

An active agent, especially sirolimus or paclitaxel, itself is no warrant for an optimal prophylaxis of restenosis. The active agent-eluting catheter balloon has to meet the requirements in its entirety. Besides the determination of dosing the active agent elution has to be effective during the short time of dilatation (around 30 sec.). The active agent elution does not depend only on the physical and chemical properties of the active agent but depends also on the properties of the utilized matrix and the interactions of the matrix and the active agent.

The inventive balloon coating ensures that the at least one an antiproliferative, immunosuppressive, anti-angiogenic, anti-inflammatory, and/or anti-thrombotic agent, preferably sirolimus or paclitaxel, is released directly and clearly to the vessel wall during balloon inflation because the active agent in the coating is approximate to the surface of the coating. The active agent is immediately and clearly purer and highly concentrated when brought into contact to the vessel wall.

The clinical benefit is the purer drug delivery, leading to a significantly higher bioavailability in arterial tissues having less unwanted side effects. The inventive coating is compared to the coating made from alcoholic solutions less sticky so that the transfer to the vessel wall is more uniform having less residuals on the balloon after dilatation. The use of a water soluble shellac salt such as Aqualacca^{®} or Aquagold^{®} enables manufacture of a more homogenous coating which causes a homogenous transfer and a homogenous release of the active agent to the area of the lesion site. This higher drug concentration in the tissue of the vessel wall provides increased effectiveness against migration and proliferation of vascular muscle cells towards the lumen of the artery at the site of the treated stenosis (lesion site). Neointimal hyperplasia is more effectively suppressed.

Materials used for the balloon catheter are all common materials, wherein the following polymers are particularly preferred: polyamides, block copolymers of polyamide, polyether and polyester, polyurethanes, polyesters and polyolefins.

The catheter balloon of the inventive catheter can be dilatable or expandable and is most preferably an angioplasty catheter balloon which could be used without crimped stent or with a crimpled stent. As stent, all kinds of common stents, such as self-expandable stents, not self-expandable stents, metal stents, polymer stents, biodegradable stents, bifurcation stents, uncoated (bare) stents, polymer coated stents, drug release coated stents, stents with a pure active agent coating etc. can be used.

Moreover, the stent can be crimped on the catheter balloon before the inventive coating procedure is carried out so that catheter balloon and stent are coated together with a "Shellaqua"-coating. However, it is preferred to use the coated balloon catheter of the present invention without stent.

The provided balloon catheter contains normally a multifold catheter balloon which will also be coated under or within the folds. Moreover it is possible to selectively coat or fill the folds. The coating within or under the folds has the advantage that during insertion of the balloon catheter the coating and thus the active agent is protected against being washed off by the blood stream.

Furthermore, the catheter balloon of the inventive balloon catheter can be coated in its expanded (inflated) or deflated state. Any commercially available dilatable catheter balloon may be used as catheter balloon. Preferably, so called multifold balloons are used, as described for example in the international patent application WO 94/23787 A1 by David H. Rammler, Labintelligence, USA; or the international patent application WO 03/059430 A1 by Scimed Life Sciences, Inc., USA; or the international patent application WO 2004/028582 A1 by Prof. Dr. Ulrich Speck or the European Patent No. EP 0519063 B1 by Medtronic Inc., USA.

Such balloons are provided with folds or wings forming essentially closed cavities when the balloon is in its compressed state but bending outward during dilatation and being capable of releasing substances contained in the folds or respectively of pressing said substances against the vessel wall.

Such balloons are advantageous since the substances enclosed in the folds or respectively active agent enclosed in the folds is protected from being detached too soon during the insertion of the catheter.

The catheter balloons according to the invention were coated with alkali salts of different commercial grades of shellac as well as with varying batches, which differed in the Lac insects, and host tree types used as well as in the time of harvest. There were no differences in release of the active agents observable in various coated catheter balloons.

Regardless of the source of shellac, "Shellaqua"-coatings of all kinds of shellac types obtained from various locations or from different insects were able to achieve the inventive results so that any kind or sort of shellac can be used in the present invention. Preferably an alkali salt of dewaxed orange shellac is used. Even more preferred an ammonium salt of dewaxed orange shellac is included in the coating on the balloon catheter.

Generally, an amount of 0.1 µg to 30 µg of the used active agent per mm² of the surface of the balloon catheter to be coated can be applied onto the surface of the balloon catheter, while an amount of 0.5 µg/mm² to 12 µg/mm² of paclitaxel and 1.0 - 15.0 µg/mm² of sirolimus is sufficient in order to achieve the desired effect on restenosis prophylaxis. The surface load of the active agent, and preferably of paclitaxel or sirolimus, on the catheter balloon is between 0.1 µg/mm² and 30 µg/mm². Preferably the amount of the active agent present on the coated balloon surface is between 1 µg/mm² and 15 µg/mm² balloon surface, more preferably between 2 µg/mm² and 10 µg/mm² and most preferably between 2.5 µg and 5 µg active agent per mm² balloon surface (µg/mm²).

Preferred is also a total amount of 10 to 1000 µg of an active agent, preferably paclitaxel or sirolimus, per catheter balloon and most preferably 20 to 400 µg per catheter balloon.

The surface load of the shellac alkali salt, preferably of shellac ammonium salt, on the catheter balloon is between 1 µg/mm² and 25 µg/mm². Preferably the amount of shellac alkali salt, preferably of shellac ammonium salt, present on the coated balloon surface is between 2.5 µg/mm² and 15 µg/mm² balloon surface.

The surface of the catheter balloon may be textured, smooth, rough, harsh, provided with cavities or provided with channels open towards the outside of the balloon. In the case, a textured surface of the catheter balloon is desired, the surface of the catheter balloon can be textured mechanically, chemically, electronically and/or by means of radiation to allow for an improved adhesion of the active agent and to assist the precipitation or crystallization of the active agent.

The content of the active agent in the active agent containing solution or in the solution of the aqueous solution of the active agent and shellac is between 1 µg to 1 mg of the active agent per ml solution, preferably between 10 µg to 500 µg of the active agent per 1 ml solution, more preferably between 30 µg to 300 µg of the active agent per 1 ml solution, and most preferably between 50 µg to 100 µg of the active agent per 1 ml solution. The content of the shellac in the aqueous shellac solution of an alkali salt is between 1 µg to 10 mg of the solution, preferably between 10 µg to 500 µg of shellac per 1 ml solution.

In another embodiment the catheter balloon is coated with "Shellaqua"-coating, wherein the weight ratio of the active agent to shellac alkali salt is from 100 : 1 to 1 : 100, preferably 95:1 to 1:95, more preferable 90:1 to 1:90, more preferable 85:1 to 1:85, further preferable 80:1 to 1:80, more preferable 75:1 to 1:75, more preferably 70:1 to 1:70, more preferable 65:1 to 1:65, more preferable 60:1 to 1:60, more preferable 55:1 to 1:55, more preferable 50:1 to 1:50, more preferable 45:1 to 1:45, more preferable 40:1 to 1:40, more preferable 35:1 to 1:35, more preferable 30:1 to 1:30, more preferable 25:1 to 1:25, more preferable 20:1 to 1:20, even more preferable 15:1 to 1:15, further preferable 10:1 to 1:10 and most preferable 5:1 to 1:5.

According to the invention, the balloon catheter does not have to be completely coated. Partial coating of the catheter balloon or partial loading of certain texture elements onto the surface of the catheter balloon may be sufficient. A special catheter balloon including micro-needles or micro-pores or micro-chambers is disclosed in the international patent application no. WO 02/043796 A2 issued to Scimed Life Systems, Inc., USA, wherein inflatable and textured areas are present on the balloon surface. In said embodiment, loading or inflating certain portions of the balloon surface would be sufficient to achieve the desired therapeutic success, wherein it is also possible, evidently, that the whole surface is coated.

An especially preferred embodiment of the present invention is directed to a balloon catheter with the "Shellaqua"-coating wherein the coating comprises a concentration gradient of the active agent in direction to the balloon surface so that on top of the coating almost 100% by weight active agent is present and directly on the surface of the balloon almost 100% by weight shellac alkali salt is present while the concentration of the active agent in the shellac alkali salt decreases from 100% by weight from the top of the coating to 0% by weight directly on the surface of the balloon.

In addition to this vertical concentration gradient which is perpendicular to the longitudinal axis of the catheter balloon, a horizontal concentration gradient could be present in a further preferred embodiment. Such a horizontal concentration gradient means that in the middle of the catheter balloon the highest concentration of the active agent is present and this concentration of the active agent will decrease in proximal and also in distal direction so that the lowest active agent concentration is present at the proximal and distal ends of the catheter balloon.

Since the "Shellaqua"-coating is hard to characterize, the present invention relates also to coated balloon catheters obtained according to the inventive coating methods disclosed herein as well as to balloon catheter and dilatation catheter comprising such a catheter balloon. In comparison to coatings prepared using alcoholic solutions of shellac the stability of the release kinetics from this coating is increased and the polymeric film on the balloon catheter has better mechanical properties. For example it is less sticky.

Such balloon catheters or catheter balloons which are coated according to the invention are preferably used for treating constricted vessel segments, particularly of blood vessels and for the treatment and prophylaxis of stenosis, restenosis, arteriosclerosis and fibrotic vessel constriction. Furthermore the coated balloon catheters of the present invention are suitable for dilatation in patients (for example patients on hemodialysis) with failing arteriovenous fistulas (AV-shunts).

Balloon catheter or catheter balloons which are coated according to the invention are preferably suited for the treatment and prophylaxis of in-stent restenosis, i.e. a reoccurring vessel constriction within an already implanted stent. Furthermore, the catheter balloons coated according to the invention are particularly suited for the treatment of small vessels, like coronary arteries, preferably such vessels having a vessel diameter of less than 2.5 mm. But also treatment of larger vessels with a vessel diameter up to 8 mm, like the treatment of femoro or popliteal artery lesions, is possible.

The balloon catheters coated according to the invention are preferably used in the cardiovascular area, but the catheter balloons coated according to the invention are also suited for the treatment of peripheral blood vessels, vessel constrictions of biliary tracts, esophagus, urinary tracts, pancreas, renal tracts, pulmonary tracts, trachea, small intestine and large intestine.

Furthermore, a second active agent may be added to the active agent containing solution. Said further active agent can be selected from the following group comprising or consisting of:
abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-AII, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids and derivatives thereof, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, β-lapachone, podophyllotoxin, podophyllic acid-2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, S-nitroso derivatives, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel and derivatives thereof, 6-α-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), rapamycin derivatives, biolimus A9, pimecrolimus, everolimus, zotarolimus, tacrolimus, fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

Due to the inventive coating method, the active agent-shellac alkali salt composite dried at the surface of the catheter balloon has a special consistence, which is hard to characterize but seems to be essential for the optimized drug release and local transfer into the cell wall of the lesion segment and the incorporation, especially into the smooth muscle cells. Thus the improved structure of the "Shellaqua"-coating has direct impact of the antiproliferative effect of the balloon catheter coated according to the solution.

Another aspect of the present invention are balloon catheter comprising a "Shellaqua"-coating wherein the coating comprises further a water soluble polymer and/or a plasticizer.

Basically water soluble polymers are highly hydrophilic as a result of the presence of oxygen and nitrogen atoms: hydroxyl, carboxylic acid, sulfonate, phosphate, amino, imino groups etc.. Water soluble polymers as herein are preferably macromolecules such as naturally occurring biopolymers such as polysaccharides and polypeptides as well as semi-synthetic derivatives thereof but also completely synthetically prepared compounds.

Thereby it is preferred that the water soluble polymer is selected from the group comprising cellulose, hydroxypropyl methylcellulose (HPMC), hydroxypropyl cellulose (HPC), carboxymethyl cellulose (CMC), polyvinylpyrrolidone (PVP), starch, hydroxyl ethyl starch, polyacrylic acid, polyethyleneimine, dextran, agar, carrageenan, alginate, copolymers and/or mixtures of these substances. Addition of sodium alginate, hydroxypropyl methylcellulose and polyvinylpyrrolidine result in increased solubility of the obtained coatings.

The term "plasticizers" as used herein refers to substances added to a coating or coating solution in order to modify their physical properties, like imparting viscosity, flexibility, or softness. Their uses include also preventing dried coatings from becoming too brittle.

Thereby it is preferred that the plasticizers are chosen from the group consisting of glycerine, propylene glycol, mineral oil, triacetin, polyethylene glycol, glyceryl monostearate, acetylated monoglyceride, polysorbate, oleic acid, butyryl-trihexylcitrat (BTHC), and glyceryl tricaprylate/caprate.

Another aspect of the present invention is a balloon catheter comprising a "Shellaqua"-coating wherein the coating comprises further a fatty acid and preferably an unsaturated fatty acid.

Preferred fatty acids are selected from the following group: octanoic acid (caprylic acid), decanoic acid (capric acid), dodecanoic acid (lauric acid), tetradecanoic acid (myristic acid), hexadecanoic acid (palmitic acid), heptadecanoic acid (margaric acid), octadecanoic acid (stearic acid), eicosanoic acid (arachidic acid), docosanoic acid (behenic acid), tetracosanoic acid (lignoceric acid), cis-9-tetradecenoic acid (myristoleic acid), cis-9-hexadecenoic acid (palmitoleic acid), cis-6-octadecenoic acid (petroselinic acid), cis-9-octadecenoic acid (oleic acid), cis-11-octadecenoic acid (vaccenic acid), cis-9-eicosenoic acid (gadoleic acid), cis-11-eicosenoic acid (gondoic acid), cis-13-docosenoic acid (erucic acid), cis-15-tetracosenoic acid (nervonic acid), t9-octadecenoic acid (elaidic acid), t11-octadecenoic acid (t-vaccenic acid), t3-hexadecenoic acid, 9,12-octadecadienoic acid (linoleic acid), 6,9,12-octadecatrienoic acid (γ-linoleic acid), 8,11,14-eicosatrienoic acid (dihomo-γ-linolenic acid), 5,8,11,14-eicosatetraenoic acid (arachidonic acid), 7,10,13,16-docosatetraenoic acid, 4,7,10,13,16-docosapentaenoic acid, 9,12,15-octadecatrienoic acid (α-linoleic acid), 6,9,12,15-octadecatetraenoic acid (stearidonic acid), 8,11,14,17-eicosatetraenoic acid, 5,8,11,14,17-eicosapentaenoic acid (EPA),7,10,13,16,19-docosapentaenoic acid (DPA), 4,7,10,13,16,19-docosahexaenoic acid (DHA), 5,8,11-eicosatrienoic acid (mead acid), 9c 11t 13t eleostearic acid, 8t 10t 12c calendic acid, 9c 11t 13c catalpic acid, 4, 7, 9, 11, 13, 16, 19 docosaheptadecanoic acid (stellaheptaenoic acid), taxoleic acid, pinolenic acid, sciadonic acid, 6-octadecynoic acid (tariric acid), t11-octadecen-9-ynoic acid (santalbic or ximenynic acid), 9-octadecynoic acid (stearolic acid), 6-octadecen-9-ynoic acid (6,9-octadecenynoic acid), t10-heptadecen-8-ynoic acid (pyrulic acid), 9-octadecen-12-ynoic acid (crepenynic acid), t7,t11-octadecadiene-9-ynoic acid (heisteric acid), t8,t10-octadecadiene-12-ynoic acid, 5,8,11,14-eicosatetraynoic acid (ETYA), eleostearic acid, calendic acid, catalpic acid, stellaheptaenoic acid, taxoleic acid, retinoic acid, isopalmitic acid, pristanic acid, phytanic acid, 11,12-methyleneoctadecanoic acid, 9,10-methylenhexadecanoic acid, coronaric acid, (R,S)-lipoic acid, (S)-lipoic acid, (R)-lipoic acid, 6,8-bis(methylsulfanyl)-octanoic acid, 4,6-bis(methylsulfanyl)-hexanoic acid, 2,4-bis(methylsulfanyl)-butanoic acid, 1,2-dithiolane carboxylic acid, (R,S)-6,8-dithiane octanoic acid, (R)-6,8-dithiane octanoic acid, (S)-6,8-dithiane octanoic acid, cerebronic acid, hydroxynervonic acid, ricinoleic acid, lesquerolic acid, brassylic acid and thapsic acid and mixtures thereof.

Preferably the unsaturated fatty acids are chosen from the following group: cis-9-tetradecenoic acid (myristoleic acid), cis-9-hexadecenoic acid (palmitoleic acid), cis-6-octadecenoic acid (petroselinic acid), cis-9-octadecenoic acid (oleic acid), cis-11-octadecenoic acid (vaccenic acid), cis-9-eicosenoic acid (gadoleic acid), cis-11-eicosenoic acid (gondoic acid), cis-13-docosenoic acid (erucic acid), cis-15-tetracosenoic acid (nervonic acid), t9-octadecenoic acid (elaidic acid), t11-octadecenoic acid (t-vaccenic acid), t3-hexadecenoic acid, 9,12-octadecadienoic acid (linoleic acid), 6,9,12-octadecatrienoic acid (γ-linoleic acid), 8,11,14-eicosatrienoic acid (dihomo-γ-linolenic acid), 5,8,11,14-eicosatetraenoic acid (arachidonic acid), 7,10,13,16-docosatetraenoic acid, 4,7,10,13,16-docosapentaenoic acid, 9,12,15-octadecatrienoic acid (α-linoleic acid), 6,9,12,15-octadecatetraenoic acid (stearidonic acid), 8,11,14,17-eicosatetraenoic acid, 5,8,11,14,17-eicosapentaenoic acid (EPA),7,10,13,16,19-docosapentaenoic acid (DPA), 4,7,10,13,16,19-docosahexaenoic acid (DHA), 5,8,11-eicosatrienoic acid (mead acid), 9c 11t 13t eleostearic acid, 8t 10t 12c calendic acid, 9c 11t 13c catalpic acid, 4, 7, 9, 11, 13, 16, 19 docosaheptadecanoic acid (stellaheptaenoic acid), taxoleic acid, pinolenic acid, sciadonic acid, 6-octadecynoic acid (tariric acid), t11-octadecen-9-ynoic acid (santalbic or ximenynic acid), 9-octadecynoic acid (stearolic acid), 6-octadecen-9-ynoic acid (6,9-octadecenynoic acid), t10-heptadecen-8-ynoic acid (pyrulic acid), 9-octadecen-12-ynoic acid (crepenynic acid), t7,t11-octadecadiene-9-ynoic acid (heisteric acid), t8,t10-octadecadiene-12-ynoic acid, and 5,8,11,14-eicosatetraynoic acid (ETYA) and mixtures thereof.

The mixtures comprise especially mixtures of the pure unsaturated compounds. Omega-3 as well as omega-6 fatty acids are especially preferred.

The following examples illustrate potential embodiments of the invention without limiting the scope of the invention to said precise examples.

### Description of the Figure

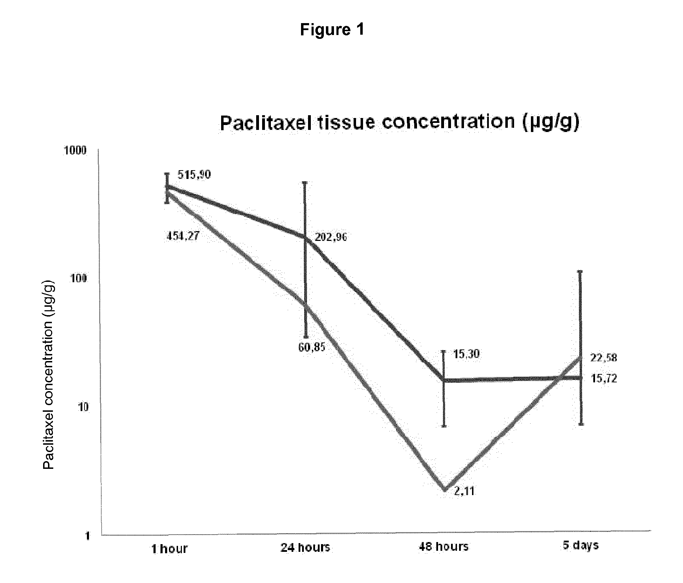

Figure 1: shows the intramural Paclitaxel concentration in [µg/g] obtained after dilatation of catheter balloons with a "Shellaqua"-coatingaccording to the invention (see Example 9)

### Examples

### Example 1 Coating of a catheter balloon with paclitaxel and AQUALACCA 25

Firstly, 120 mg paclitaxel are solved in 800 µL ethanol and mixed with 800 µL of AQUALACCA 25 by stirring for 24 h at room temperature.

The AQUALACCA 25 (which is a water soluble shellac ammonium salt) solution is applied to the surface of a folded balloon which is rotatably mounted by a pipetting device. Then the folded balloon is dried under slow rotation at room temperature. The paclitaxel solution is then sprayed on the balloon catheter in a way that 3.0 µg/mm² paclitaxel are applied. Then the balloon is dried without rotation at room temperature. Finally, the AQUALACCA 25 is applied as a separate topcoat by a pipetting device on the active agent layer. 1µg/mm² top coat is applied. Subsequently, the catheter balloon is thoroughly dried for 30 minutes at 50 °C. The presence of a stent or drug-eluting stent crimped on the balloon does not interfere with the coating process.

### Example 2 Coating of a catheter balloon with a "Shellaqua"-coating containing sirolimus

A commercially available dilatation catheter with expandable balloon made of a polyamide is provided. The balloon surface is textured but without channels or cavities.

Ground shellac was dissolved in 2.5% (w/w) ammonium bicarbonate solution at 40°C under continuous mechanical stirring to produce a final concentration of 20% (w/w). The solution was heated up to 70°C for 30 minutes under continuous stirring, to evaporate excessive ammonium in order to reach the optimum pH 7.3. Then water was added to achieve the concentration of 20% (w/w).

Subsequently this solution was applied onto the horizontal area of the surface of the catheter balloon by brushing. A solution of 140 µg of rapamycin in 2.0 mL of water is prepared and the catheter balloon is immersed into said solution. Subsequently, the catheter balloon is thoroughly dried and sterilized with ethylene oxide.

### Example 3 Coating of a catheter balloon with a "Shellaqua"-coating containing sirolimus and gum arabic

A balloon of a balloon catheter suitable for expansion vessel dilatation is degreased with acetone and ethanol in an ultrasonic bath for 10 minutes and the balloon catheter is then dried at 100 °C. Solution of gum arabic was prepared by adding the spray-dried powder to 1% (w/w) ammonium bicarbonate solution in demineralised water at 50°C and stirring mechanically until the gum was dissolved completely. Ammonium bicarbonate was added until increase of the pH of the gum solution to above 7. Subsequently this solution was mixed with shellac so that 18% w/w solutions were prepared. 120 mg sirolimus are solved in 1 mL aqueous shellac solution and is applied to the catheter balloon by spraying. The coated catheter balloon is dried within 13 hours at 70 °C.

### Example 4 Coating of a catheter balloon with a "Shellaqua"-coating containing paclitaxel and a plasticizer

Firstly, 120 mg paclitaxel are solved in 800 µL ethanol and 190 g shellac and 9 g glycerol are solved in 1000 mL 2.5% (w/w) ammonium bicarbonate solution stirring for 24 h at 40°C. After this 100 µL of the solution of paclitaxel is mixed with 900 µL of the shellac ammonium salt solution and pipetted on a catheter balloon. The coated catheter balloon was dried over night at 70 °C.

### Example 5 Coating of a catheter balloon with a "Shellaqua"-coating containing sirolimus using a gradient mixer

A solution of rapamycin and a solution of shellac salt were prepared as described in Example 2. After this 100 µL of the solution of sirolimus is mixed with 900 µL of the shellac salt solution.

The pure shellac salt solution is applied to the surface of a partially unfold balloon which is rotatably mounted by a spraying device. Then the balloon is dried under slow rotation at room temperature. The base coat contained 1 µg/mm² shellac salt on the balloon surface.

The solution containing sirolimus and shellac is poured in the first chamber of a gradient mixer and the pure sirolimus solution is poured in the second, posterior chamber. The outlet of the gradient mixer is connected to a spray gun. The solution out of the gradient mixer is then sprayed on the balloon catheter with the base coat in a way that increasing sirolimus concentration is applied. A total of 3.0 µg/mm² sirolimus is applied. Then the balloon is dried under slow rotation at room temperature.

### Example 6 Coating of a catheter balloon with a "Shellaqua"-coating containing sirolimus

A commercially available dilatation catheter with expandable balloon made of a polyamide is provided. The balloon surface is textured but without channels or cavities.

Ground shellac was dissolved in 2.5% (w/w) sodium bicarbonate solution at 40°C under continuous mechanical stirring water was added to achieve the concentration of 20% (w/w). Subsequently this solution was applied onto the horizontal area of the surface of the catheter balloon by brushing. A solution of 140 µg of rapamycin in 2.0 mL of water is prepared and the catheter balloon is immersed into said solution. Subsequently, the catheter balloon is thoroughly dried and sterilized with ethylene oxide.

### Example 7 Coating of a catheter balloon with a "Shellaqua"-coating containing sirolimus

Firstly, 100 mg sirolimus are solved in 1 mL of AQUALACCA 25.

The AQUALACCA 25solution containing sirolimus is applied to the surface of an unfold balloon which is rotatably mounted by spraying. Then the balloon is dried under slow rotation at room temperature. Thereafter a second layer of the same coating solution is sprayed as described before. Subsequently, the catheter balloon is thoroughly dried for 2 hours at 50 °C. Finally, 5.0 µg/mm² balloon surface sirolimus were applied.

### Example 8 Coating of a catheter balloon with a "Shellaqua"-coating containing paclitaxel

Firstly, 120 mg palitaxel are solved in 1 mL of AQUAGOLD. The water of this solution is evaporated under vacuum and the pellet is resolved in 1 mL ethanol.

The resulting solution containing paclitaxel is applied to the surface of a multifold balloon which is rotatably mounted by spraying. Then the balloon is dried under slow rotation at room temperature. Thereafter a second and a third layer of the same coating solution is sprayed as described before. Subsequently, the catheter balloon is thoroughly dried for 2 hours at 50 °C. Finally, 4.0 µg/mm² balloon surface paclitaxel were applied.

### Example 9: Pharmacokinetic Evaluation of balloons coated according to the invention

This study sough to evaluate short-term (1 hour - 5 days) paclitaxel tissue uptake and retention delivered via the inventive catheter balloons.

Eight polish domestic pigs of 34-43 kg body weight were included in the study in which 24 paclitaxel eluting balloons were deployed. Procedures were carried out Center for Cardiovascular Research and Development, American Heart of Poland Inc, between July and August of 2013. The appropriate approval of regional Bioethical Committee was obtained. The three coronary arteries (LAD, LCX, RCA) of each animal were randomly assigned in 5:1 ratio to study groups.

The studied catheters with the following coatings were evaluated:
Group 1. 3.0 µg/mm² Paclitaxel + 3.0 µg/mm² Shellac salt (AQUALACCA 25)
Group 2. 3.0 µg/mm² Paclitaxel + 2.0 µg/mm² Shellac salt (AQUALACCA 25)

All studied balloons were 3.0 mm in diameter and 15 mm length.

### Methods

All animals received dual antiplatelet therapy consisting of oral acetylsalicylic acid (325mg initial dose and 75mg in next days) and clopidogrel (300mg initial dose and 75mg subsequently) starting three days prior to intervention and continuing until sacrifice. After anesthesia induction with propofol, the animals were intubated and supported with mechanical ventilation. A propofol continuous infusion was started to maintain a surgical plane of anesthesia. Subsequently, an arterial sheath was introduced to the left or right femoral artery utilizing percutaneous Seldinger technique. An initial bolus of heparin (~ 400 U/kg) was administered and ACT was measured every 30 minutes to maintain ACT time of at least 300 seconds. Coronary angiograms were performed after administration of intracoronary nitroglycerin (200µg). The selection of the target site was made based on visual assessment of anatomy and quantitative coronary angiography (QCA) analysis. These sites were chosen for the avoidance of side branches and segments with tapering greater than 10% to ensure uniform interaction of the stent coating with the arterial wall. The injury balloon was then inflated at a steady rate to a pressure sufficient to achieve a balloon to artery ratio of 1.2-1.3:1.0. Following the injury procedure, the treatment balloon was advanced in the same location and inflated at similar balloon to artery ratio for 60 seconds. At pre-determined time points the animals were euthanized utilizing approved euthanasia solutions. The hearts were harvested as quickly as possible after euthanasia, using precautions to avoid damage to the study vessels. The hearts were examined for abnormal findings and were labeled with the animal identification number, protocol number and date of collection. The hearts were flushed with heparanized saline until cleared of blood. The studied segments were dissected under stereoscopic microscopy guidance, using coronary angiography and side branches as landmarks. All study vessel segment were labeled with the animal identification number, protocol number and date of collection. All tissues were placed in containers, frozen in dry ice in -68 C and sent to the HPLC test site.

### Qualitative Coronary Angiography

Coronary arteries angiographies were obtained using Siemens Coroskop Millenium Edition angiographic unit. Judkins Right, 6 French guiding catheter was utilized to obtain coronary angiography. QCA analysis was performed in a blinded fashion utilizing QAngio XA Software version 7.1.14.0 (Medis Medical Imaging Systems) from two contralateral projections. The baseline and 28-day follow-up reference vessel diameters (RVD) were taken from the proximal and distal portion of the treated segments using the guiding catheter as a standard for measurement. The balloon-to artery ratio was calculated. Percent diameter stenosis (%DS) at follow-up was calculated as: [1-(MLD/RVD)] x 100%.

### HPLC Analysis

The paclitaxel concentration of plasma, LAD, LCx and RCA were measured by high-performance liquid chromatography (AnaKat Institut für Biotechnologie GmbH, Berlin, Germany, analysis blinded to sample origin). Briefly, after thawing, the tissues were weighed at ambient temperature and, depending on the weights, different volumes of ethanol were added to the samples (sufficient ethanol to cover the tissue completely). Then, samples were treated with ultrasound for 40 min and about 200 ml samples were centrifuged. A calibration line was produced in the range between 50 and 5000 ng/ml. The samples for the calibration line were prepared by dilution of a stock solution with a concentration of 1000 mg/ml. Aliquots of all samples (samples from tissue and calibration line) were transferred into autosampler vials and the same volume of 0.1% formic acid was added. The flow rate of the high-performance liquid chromatography system was 0.2ml/min through a column of ODS Hypersil (ThermoElectron Corporation, Thermo Scientific, Waltham, Massachusetts, USA), particle size 5 m, pore size 120A°. The isocratic mobile phase consisted of 70% methanol containing formic acid (0.1%). Paclitaxel was detected by mass spectrometry in multiple reaction monitoring mode with a transition of paclitaxel from 854 to 105AMU. The tissue paclitaxel concentration was expressed in µg/g.

### Follow - up

The animals were scheduled for 1, 24, 48 hours, 5 days (2 pigs per each time period) according to study scheme presented in table 1

**Table 1 study scheme showing distribution of the catheter balloons to the vessels and animals**

| **Follow up** | **Animal Number** | **Tested balloons** | | |
|---|---|---|---|---|
| | | **RCA** | **LAD** | **LCX** |
| 1 hour | 1 | Group 1 | Group 1 | Group 1 |
| 1 hour | 2 | Group 1 | Group 1 | Group 2 |
| 24 hours | 3 | Group 1 | Group 1 | Group 1 |
| 24 hours | 4 | Group 1 | Group 2 | Group 1 |
| 48 hours | 5 | Group 1 | Group 1 | Group 1 |
| 48 hours | 6 | Group 2 | Group 1 | Group 1 |
| 5 days | 7 | Group 1 | Group 1 | Group 1 |
| 5 days | 8 | Group 1 | Group 1 | Group 2 |

### Statistical analysis

Results are expressed as median and interquartile ranges. Because of a limited number of samples in the group 2 (only one per time point) no statistical tests were applied.

### RESULTS

### Pre-Operative Procedures

After an overnight fast, the animals were pre anesthetized with a mixture based on body weight. These drugs include: Atropine (1 mg / 20 kg sc.), Ketamine (1 ml/10 kg i.m.) and Xylazine (1 ml/10 kg im). The injection was given intramuscularly in either the neck or rear muscle quadrant by a qualified animal technologist. The animal was transferred to the preparation room, where an intravenous line was placed in the auricular marginal vein, and intravenous fluids (lactated ringers or 0.9% saline) were administered throughout the procedure. Anti-arrhythmics were added to these IV fluids (Lidocaine 200mg/liter, Metoprolol 5mg/liter) if necessary. When the animal reached an adequate anesthetic state (with a propofol bolus), it was intubated with an appropriate size endotracheal tube, which was tied into place and the cuff inflated to prevent leakage. The animal was then transferred to the cath lab, placed on the table and attached to the anesthesia and ventilator unit.

### Procedures

The vessel injury involved inflation of regular angioplasty balloon at 1.2-1.3:1.0 balloon-to-artery ratio (live QCA analysis) in previously selected arterial segment in order to achieve an appropriate overstretch and injury. For predilation, all balloons were inflated for 30s.
Then, a total of 24 tested balloons were inflated: twenty catheter balloons of group 1 and four catheter balloons of group 2, as shown in Table 1. Each of them was inspected before delivery. No signs of structure abnormality were noticed. The coating was not visible. Balloons were easily introduced into the selected arterial segment through femoral artery access and successfully deployed in previously injured segment. The tested balloons were inflated for 60s, except one balloon which burst after 25 seconds. Due to absence of anatomical landmarks, bare metal stents were implanted distally to treated segments in two cases (stent Apollo S 2,25mmx19mm).

### Baseline vessel and balloon deployment characteristics

There were no differences in the baseline QCA parameters such as vessels baseline proximal and distal reference diameter and average vessel diameter in the whole group as well as within each time period (Table 2). The average overstretch was 120 - 130% and was reproducible among groups. All tested balloons were 3.0 mm in diameter and 15 mm length and stayed in circulation for 3 minutes ± 20 seconds.

**Table 2 Baseline QCA vessel characteristics**

| Timepoint | RVD [mm] | Injury balloon [mm] | PCB diameter [mm] | Overstretch |
|---|---|---|---|---|
| | Median (IQR) | Median (IQR) | Median (IQR) | (%) |
| 1h (n=6) | 2.3 | 2.96 | 2.90 | 129 |
| | (2.23; 2.3) | (2.90; 3.02) | (2.87; 2.90) | |
| 24h (n=6) | 2.4 | 2.93 | 2.83 | 122 |
| | (2.31; 2.56) | (2.90; 3.54) | (2.74; 3.08) | |
| 48h (n=6) | 2.3 | 2.78 | 2.76 | 121 |
| | (2.26; 2.4) | (2.71; 2.91) | (2.68; 2.86) | |
| 5d (n=6) | 2.38 | 2.91 | 2.86 | 123 |
| | (2.28; 2.44) | (2.90; 3.06) | (2.62; 3.07) | |

### Paclitaxel concentration analysis

### Paclitaxael tissue uptake and retention

Within the follow-up period, there were no deaths or major adverse events cardiac events were noted. All animals remained in good general condition until euthanasia. At one hour follow up, the inventive catheter balloons delivered paclitaxel in a concentration of 454.27 µg/g and 515.9 µg/g, respectively. At one day follow up in the group 1, paclitaxel median concentration found in the vessel was 202.96 µg/g, whereas the second balloon set for the same follow up delivered 60,85 µg/g. The similar trend was observed after 48 hours (15.3 vs. 2.11 µg/g, respectively). In the final observation, paclitaxel concentration was comparable in both groups (Figure 1). One balloon of group 1 did not deliver any drug to the vessel wall at 5 day follow-up.

### Paclitaxel residuals on balloon

The analysis of paclitaxel residuals on the balloon, showed that nearly 50% of the baseline drug amount was left on the surface of group 2 balloons and 40% in group 1 as shown by the HPLC analysis.

### Conclusions

All tested balloons were easily introduced and deployed at study sites. No delivery or withdrawal problems occurred. The balloon diameters at nominal inflation reached their designed diameter. No adverse events were noted, neither directly after procedures nor at follow-up. On autopsy no macroscopic signs of myocardial infarction, or inflammation within studied site were noted. In the vessels designated for 5 days follow up, adhesion around treated vessel segments were observed which could be caused either by the injury or drug toxicity. It must be noted, that due to very short term of observation and design of the study, the safety of studied balloon catheters could not be established.
The baseline studied vessel characteristics between groups were similar with regard to reference diameter and overstretch (130%). Except one balloon, all inflations were performed for 60 s and all balloon remained within the same period of time in circulation. Both tested paclitaxel balloons delivered paclitaxel to the vessel wall. In all vessels after 1-hour paclitaxel was found in the range between 360-1135 µg/g, therefore proving deliverability of drug into the wall. It seems that group 1 balloon delivers paclitaxel in higher concentration, however due to low sample number, the significance of this finding remains inconclusive and hypothetical. At 5 days follow up the group 1 has shown significant tissue retention; however the result were variable (0-105 microgram) which is typical for this type of technology. Thus this proof-of-principle study showed that the inventive devices allow for the accumulation of therapeutic active agent concentrations in the arterial wall for at least 5 days post deployment.

### Example 10: Biological test of prior art balloon catheters

Eight polish domestic pigs of 35-42 kg body weight were included in the study in which 24 paclitaxel eluting balloons were deployed. The appropriate approval of regional Bioethical Committee was obtained. The three coronary arteries (LAD, LCX, RCA) of each animal were randomly assigned in 1:1:1 ratio to either study group.

Three studied catheters with the following coatings were evaluated:
1. 3.0 µg/mm² Paclitaxel + 0.3 µg/mm² Alpha Linolen + 0.3 µg/mm² Boswellic acid
2. 3.0 µg/mm² Paclitaxel + 0.3 µg/mm² z Alpha Linolen
3. 3.0 µg/mm² Paclitaxel and 3.0 µg/mm² shellac applied as ethanolic solution (shellac in its acid form; balloon of the prior art)

All studied balloons were 3.0 mm in diameter and 20 mm length.

### Methods

Animals received antiplatelet therapy consisting of acetylsalicylic acid and clopidogrel three days prior to intervention and throughout the study. Under general anesthesia femoral artery access through 6 F sheath was gained for stent introduction and implantation into the two different coronary arteries. All balloons were implanted under "live" quantitative angiography analysis guidance at an inflation pressure which ensured a balloon/artery diameter ratio of 1.15 : 1.0.

The Quantitive Coronary Angiography (QCA) analysis was performed with the use of CMS-QCA software (Medis) and angiograms were recorded in DICOM format. Two contralateral projections were chosen for stent assessment. At pre-determined time points the animals were euthanized. The hearts were harvested as quickly as possible after euthanasia, using precautions to avoid damage to the study vessels. The hearts were examined for abnormal findings and were labeled with the animal identification number, protocol number and date of collection. The hearts were flushed with normal saline until cleared of blood and then pressure-perfusion fixed at 80-100 mmHg with 10% neutral buffered formalin (NBF). Samples of abnormal tissues were collected and undergo immersion fixation with 10% NBF. All study vessel segment were labeled with the animal identification number, protocol number, tissue types and date of collection. All tissues were placed in containers and frozen in dry ice in -68 C and sent to the HPLC test site. The heart for each animal was placed in its own separate container.

### HPLC Analysis

The paclitaxel concentration of plasma, LAD, LCx and RCA were measured by high-performance liquid chromatography (AnaKat Institut für Biotechnologie GmbH, Berlin, Germany, analysis blinded to sample origin). Briefly, after thawing, the tissues were weighed at ambient temperature and, depending on the weights, different volumes of ethanol were added to the samples (sufficient ethanol to cover the tissue completely). The samples were then treated with ultrasound for 40 min. About 200 ml samples were centrifuged.
A calibration line was produced in the range between 50 and 5000 ng/ml. The samples for the calibration line were prepared by dilution of a stock solution with a concentration of 1000 mg/ml. Aliquots of all samples (samples from tissue and calibration line) were transferred into autosampler vials and the same volume of 0.1 % formic acid was added. The flow rate of the highperformance liquid chromatography system was 0.2 ml/min through a column of ODs Hypersil (ThermoElectron Corporation, Thermo Scientific, Waltham, Massachusetts, USA), particle size 5 m, pore size 120A°. The isocratic mobile phase consisted of 70% methanol containing formic acid (0.1%). Paditaxel was detected by mass spectrometry in multiple reaction-monitoring mode with a transition of paclitaxel from 854 to 105AMU. The tissue paclitaxel concentration was expressed in µg/g.

### Pre-Operative Procedures

After an overnight fast, the animals were pre anesthetized with a mixture based on body weight. These drugs include: Atropine (1 mg / 20 kg sc.), Ketamine (4 mL / 10 kg im.) and Xylazine (1 ml / 10 kg im). The injection was given intramuscularly in either the neck or rear muscle quadrant by a qualified animal technologist. The animal was transferred to the preparation room, where an intravenous line was placed in the auricular marginal vein, and intravenous fluids (lactated ringers or 0.9% saline) were administered throughout the procedure. Anti-arrhythmics were added to these IV fluids (Lidocaine 200mg/liter, Metoprolol 5mglliter). When the animal reached an adequate anesthetic plane (gas mask with 1-3% isoflurane), it was intubated with an appropriate size endotracheal tube, which was tied into place and the cuff inflated to prevent leakage. The animal was then transferred to the cath lab, placed on the table and attached to the anesthesia and ventilator unit.

### Procedures

In total 24 balloons were deployed, eight of group 1 and 2 and 8 of group 3 (according to the invention). Each of them was inspected before delivery. No signs of structure abnormality were noticed. Balloons were easily introduced into the selected arterial segment through femoral artery access and successfully deployed in the desired segment after live QCA guidance to ensure balloon/artery ratio 1,1 : 1. All tested balloons were inflated for 60s. Due to overstretch in 3 case dissections post balloon inflation were visible, although the vessel remained opened and distal flow was not impaired, therefore a stent implantation was not necessary.

### Follow - up

The animals were scheduled for 1 hour, 1, 3 and 7 days (2 pigs per time period). Within the entire follow-up period, neither death nor major adverse events cardiac events were noted. All animals remained in good general condition and a steady bodyweight increase was observed.

### Statistical analysis

Results are expressed as mean and standard deviation (SD). Normal distribution of variables was verified by Kolmogorov-Smirnov test. The variance uniformity was verified with the use of Levene test. Angiographic and HPLC anaylsis data were analyzed using ANOVA tests. In case of skewed distribution or non-uniformity of variance a nonparametric Kruskal-Wallis and U Mann-Whitney tests were used. The p-value <0.05 was considered statistically significant.

### Results

Baseline vessel and balloon deployment characteristics: There were no differences in the baseline QCA results such as vessels baseline, reference diameter, minimal lumen diameter, balloon diameter and stent to artery ratio in the whole group as well as within each time period between the studied groups.

### Paclitaxel concentration analysis

There was a significantly higher intramural vessel concentration of paclitaxel in group 3 at 1 hour observation. At 1 day, although not statistically significant it remained numerically much higher. At 3 and 7 days, in the group 3, the concentration decreased to 1 µg/g and to a not recognizable level in groups 1 and 2 (Table 3). These results were sustained in percentage of initial loading dose analysis.

**Table 3. Vessel intramural paclitaxel Concentration**

| **µg/g** | **Group 3** | **Group 1** | **Group 2** | **ANOVA** |
|---|---|---|---|---|
| | **N=2** | **N=2** | **N=2** | **p** |
| **1 hour** | 43.8 ± 14.9 | 0.2 ± 0.2 | 1.3 ± 1.9 | 0.02 |
| **24 hours** | 107.6 ± 97 | 1.1 | 0 | 0.2 |
| **3days** | 4.1 ± 6 | 1.8 ± 2.5 | 1.2 ± 0.3 | 0.73 |
| **7 days** | 4.7 ± 6.6 | 0 | 0 | 0.4 |

All tested balloons were easily introduced and deployed at study sites. No delivery or withdrawal problems occurred. The balloon diameters at nominal inflation reached their designed diameter. Adverse events were noted neither after procedures nor at follow up. On autopsy no macroscopic signs of myocardial infarction, or inflammation within studied site were observed. The studied baseline vessel characteristics were similar between groups with regard to reference diameter and minimal lumen diameter. Most importantly the stent to artery ratio of 1.1:1 resulted in similar overstretch between the studied groups. All inflation was performed for 60 s and all balloon remained within the same period of time in circulation. Basing on previous studies this overstretch and inflation time should definitely provide proper and reproducible conditions for paclitaxel delivery (1,2).

### Conclusions

All tested balloons were easily introduced and deployed at study sites. No delivery or withdrawal problems occurred. Both paclitaxel balloons according to the invention (example 9) coated with a shellac ammonium salt delivered paclitaxel more effectively to the vessel wall. The paclitaxel concentration in the tissue after deployment of a catheter balloon according to the invention was approximately 10 times higher (about 500 µg/g) than using a catheter balloon coated with shellac in its acid form (prior art balloon; after 1 h about 50 µg/g) as shown in table 3, which indicates relatively poor drug in tissue concentration resulting from a coating with shellac in its acid form and also catheter balloons coated with Alpha Linolen as carrier substance.

### Example 11: Safety study of balloons coated according to the invention

Porcine coronary arteries were dilated using 3 types of drug-eluting balloon coated according to the invention (3x4 pigs) in 12 pigs, with a follow up (FUP) time of 1 h, 3h, 24h and 48h. The balloons were inflated with 1.3:1 overstretching, for 2x30 sec. After FUP periods, the arteries were explanted, stored in liquid nitrogen, and sent for tissue paclitaxel/sirolimus measurements. Ten catheter tips of each balloon, and 12-15 plasma samples (from blood sampling immediately after balloon use, 5, 10 min and 60 min post ballooning) were also sent for evaluation.

The studied catheters with the following coatings were evaluated:
Group 1. 3.0 µg/mm² Paclitaxel + 3.0 µg/mm² Aqualacca25 + 2.0 µg/mm² PEG as top coating ("Master")
Group 2. 3.0 µg/mm² Paclitaxel + 2.0 µg/mm² Aqualacca25 ("Ren")
Group 3. 5.0 µg/mm² Sirolimus + 3.0 µg/mm² Aqualacca25 + 0.5 µg/mm² omega fatty acid + 2.0 µg/mm² PEG as top coating.

All catheter balloons were coated by micropipetting.

All studied balloons were 3.0 mm in diameter and 20 mm length.

The study has been conducted in compliance with US Food and Drug Administration Good Laboratory Practice Regulations 21 CFR Part 58, Management Special.

The Quality Assurance Unit. in accordance with the Test Facility's Standard Operating Procedures (SOPs), has audited the protocol, study conduct.

### METHODS

**Table 4: Study Design**

| **ID** | **Implantation date** | **Explantation date** | **FUP** |
|---|---|---|---|
| GROUP 2-1h | May 14. 2013 | May 14. 2013 | 1h |
| GROUP 2-3h | May 14. 2013 | May 14. 2014 | 3h |
| GROUP 2-24h | May 13. 2013 | May 14. 2015 | 24h |
| GROUP 2-48h | May 13. 2013 | May 15. 2013 | 48h |
| GROUP 1-1h | May 15. 2013 | May 15. 2013 | 1h |
| GROUP 1-3h | May 14. 2013 | May 14. 2013 | 3h |
| GROUP 1-24h | May 13. 2013 | May 14. 2013 | 24h |
| GROUP 1-48h | May 13. 2013 | May 15. 2013 | 48h |
| GROUP 3-1h | May 15. 2013 | May 15. 2013 | 1h |
| GROUP 3-3h | May 14. 2013 | May 14. 2013 | 3h |
| GROUP 3-24h | May 13. 2013 | May 14. 2013 | 24h |
| GROUP 3-48h | May 13. 2013 | May 15. 2013 | 48h |

### Endpoint

Primary endpoint analysis: assessment of safety, in terms of adverse events and measurements of arterial tissue and plasma paclitaxel concentration and remnant paclitaxel on balloon surface. Any adverse event, such as mortality or "clinical event" was assessed.

### Animals

| | |
|---|---|
| Species: | *Sus scrofa* |
| Strain: | Yorkshire Pigs |
| Source: | Animal Industries |
| Age at Receipt: | Young adult |
| Weight at Intervention: | 30-40 kg |
| Number of Animals (including spares): | 12 |

### RESULTS

**Table 5: Implantation scheme**

| ID | Location-1 | Dil. pressure (atm) | Location-2 | Dil. Pressure (atm) | Location-3 | Dil. Pressure (atm) | Location-4 | Dil. Pressure (atm) | Location-5 | Dil. Pressure (atm) | Location-6 | Dil. Pressure (atm) |
|---|---|---|---|---|---|---|---|---|---|---|---|---|
| GROUP 3-1 | LCxdist | 14 | LADmid | 16 | LADdistP | 14 | LADdistD | 12 | Lcxmid | 16 | OMA | 14 |
| GROUP 3-3 | Lcxmid | 16 | Lcxdist | 14 | OMA | 14 | LADmid | 14 | Diag | 10 | | |
| GROUP 3-24 | LADmid | 16 | Diag | 8 | Lcxmid | 16 | Lcxprox | 16 | OMA | 12 | | |
| GROUP 3-48 | LADmid | 14 | LADdist | 12 | Diag | 12 | LADmid | 14 | OMA | 12 | | |
| GROUP 1-1 | Lcxmid | 16 | OMA | 14 | UMA | 14 | LADmid | 16 | LADdistPr | 16 | LADdistD | 12 |
| GROUP 1-3 | Lcxmid | 16 | UMA | 14 | OMA | 16 | Lcxdist | 12 | LADmid | 16 | LADdist | 14 |
| GROUP 1-24 | LADmid | 14 | LADd ist | 10 | Lcxmid | 16 | Lcxdist | 12 | OMA | 10 | | |
| GROUP 1-48 | Lcxmid | 14 | OMA | 12 | LADmid | 12 | LADdist | 10 | Diag | 10 | | |
| GROUP 2-1 | Lcxmid | 16 | OMA | 12 | Lcxdist | 12 | UMA | 10 | LADmid | 14 | Diag | 10 |
| GROUP 2-3 | Lcxmid | 16 | OMA1 | 12 | OMA2 | 14 | LADmid | 14 | Diag | 12 | | |
| GROUP 2-24 | Lcxmid | 16 | Lcxdist | 16 | OMA | 12 | LADdist | 12 | LADmid | 12 | | |
| GROUP 2-48 | OMA | 14 | Lcxdist | 12 | Lcxmid | 14 | LADmid | 16 | LADdist | 12 | | |

### No procedural complication occurred.

Generally, all animals received loading dose of clopidogrel (300 mg) and aspirin (250 mg) per os 1 day before the planned percutaneous coronary intervention (PCI). During the FUP, the pigs received a daily dose of 75 mg clopidogrel and 100 mg aspirin per os. Before PCI, the animals received 10 000 IU unfractionated heparin, supplemented with additional 2000 IU heparin in each hours during the implantation procedure, if necessary.

### GROUP 1 balloon

**Table 6: Arterial tissue paclitaxel concentration of GROUP 1 balloon**

| **GROUP 1 FUP** | **Paclitaxel in Tissue [µg/g]** |
|---|---|
| | |
| **1h (n=5)** | |
| **Mean±SD** | **28.79** ± 13.26 |
| | |
| **3h (n=5)** | |
| **Mean±SD** | **6.42** ± 3.55 |
| | |
| **24h (n=5)** | |
| **Mean±SD** | **4.59** ± 4.41 |
| | |
| **48h (n=5)** | |
| **Mean±SD** | **1.25** ± 1.64 |

Comment: The present study revealed a tissue paclitaxel level of mean 28.79 µg /g 1 h post dilation, which is lower than the desired tissue drug level (according to the literature). The elimination of the paclitaxel from the tissue was relatively quick, with rapid decrease of the tissue drug level after 3 h.

**Table 7: Plasma paclitaxel level of GROUP 1 balloon**

| | **Plasma level of PTx (ng/mL)** |
|---|---|
| | |
| post-PCI (n=2) | **5.24** ±1.00 |
| | |
| 10 min post-PCI (n=3) | **24.68** ±24.84 |
| | |
| 30 min post-PCI (n=3) | **6.33** ±1.11 |
| | |
| 60 min post-PCI (n=3) | **4.80** ±1.91 |

Comment: The measured plasma paclitaxel concentration was far below the toxic level, and much less than used for therapeutic aims. The elimination rate corresponded with the normal plasma half-life of paclitaxel of approximately 60 min in humans.

**Table 8: Balloon surface remnant paclitaxel level of GROUP 1 balloon**

| Catheter surface remnant paclitaxel amount of GROUP 1 balloon | **Paclitaxel Amount [µg]** |
|---|---|
| | |
| Mean±SD | **1.83**±0.71 |

Comment: Calculating 3 µg paclitaxel on the balloon surface (3 mm diameter and 20 mm length), the total amount of the paclitaxel on the balloon surface should be 565.2 µg. The balloon surface remnant paclitaxel amount was mean 1.83 µg (0.3%). Regarding the amount of the remnant paclitaxel on the balloon surface, a second dilation procedure with the same balloon (outside of the 2x30 sec) would not deliver further sufficient amount of paclitaxel into the vessel wall.

Considering the amount of tissue, plasma and remnant paclitaxel on balloon surface, it seems, that relatively high amount of paclitaxel is dissolved from the balloon surface during the balloon catheter placement; beginning with entering of the catheter into the circulation via femoral artery until balloon inflation in the coronary artery. As no procedural complication occurred, the duration of this time was approximately 30 to 60 sec.

### GROUP 2 balloon

**Table 9: Arterial tissue paclitaxel concentration of the GROUP 2 balloon**

| **GROUP 2 FUP** | **Paclitaxel in Tissue [µg/g]** |
|---|---|
| | |
| **1h (n=5)** | |
| **Mean±SD** | **11.46** ± 14.45 |
| | |
| **3h (n=5)** | |
| **Mean±SD** | **12.19** ± 10.87 |
| | |
| **24h (n=5)** | |
| **Mean±SD** | **9.96** ± 16.73 |
| | |
| **48h (n=5)** | |
| **Mean±SD** | **0.50** ± 0.94 |

Comment: This study revealed a tissue paclitaxel level of mean 11.46 µg/g 1 h post dilation, which is lower than the desired tissue drug level (according to the literature). The elimination of the paclitaxel from the tissue was relatively slow at 3 h.

**Table 10: Plasma paclitaxel level of GROUP 2 balloon**

| | **Plasma level of PTx (ng/mL)** |
|---|---|
| | |
| post-PCI (n=4) | **57.87** ±11.11 |
| | |
| 10 min post-PCI (n=4) | **10.15** ±9.24 |
| | |
| 10 min post-PCI (n=4) | **8.11** ±5.17 |
| | |
| 60 min post-PCI (n=3) | **3.98** ±1.64 |

Comment: The measured plasma paclitaxel concentration was far below the toxic level and much less than used for therapeutic aims. The elimination rate corresponded with the normal plasma half-life of paclitaxel of approximately 60 min in humans.

**Table 11: Balloon surface remnant paclitaxel level of GROUP 2 balloon**

| Catheter surface remnant paclitaxel amount of GROUP 2 balloon | **Paclitaxel Amount [µg]** |
|---|---|
| | |
| Mean±SD | **11.65** ±24.96 |

Comment: Calculating 3 µg paclitaxel on the balloon surface (3 mm diameter and 20 mm length), the total amount of the paclitaxel on the balloon surface should be 565.2 µg. The balloon surface remnant paclitaxel amount was mean 11.65 µg (2.1 %). Regarding the amount of the remnant paclitaxel on the balloon surface, a second dilation procedure with the same balloon (outside of the 2x30 sec) would not deliver further sufficient amount of paclitaxel into the vessel wall.

Considering the amount of tissue, plasma and remnant paclitaxel on balloon surface, it seems, that relatively high amount of paclitaxel is dissolved from the balloon surface during the balloon catheter placement; beginning with entering of the catheter into the circulation via femoral artery until balloon inflation in the coronary artery. As no procedural complication occurred, the duration of this time was approximately 30 to 60 sec.

### GROUP 3 measurements

**Table 12: Arterial tissue sirolimus concentration of the GROUP 3 balloon**

| **GROUP 3 FUP** | **Sirolimus in Tissue [µg/g]** |
|---|---|
| | |
| **1h (n=5)** | |
| **Mean±SE** | **954.2** ± 624.3 |
| | |
| **3h (n=5)** | |
| **Mean±SE** | **1072.3** ± 515.8 |
| | |
| **24h (n=5)** | |
| **Mean±SE** | **162.0** ± 138.7 |
| | |
| **48h (n=5)** | |
| **Mean±SE** | **12.3** ± 9.1 |

Comment: This study revealed a tissue sirolimus level of mean 954.2 µg/g 1 h post dilation, which seems to be the desired tissue drug level (according to the literature). The elimination of the sirolimus from the tissue was slow, with still relatively high level of drug at 24h and at 48h..

**Table 13: Plasma paclitaxel level of GROUP 3 balloon**

| | **Plasma level of sirolimus (ng/mL)** |
|---|---|
| | |
| post-PCI (n=3) | **1.75** ±3.51 |
| | |
| 10 min post-PCI (n=3) | **0** ±0 |
| | |
| 10 min post-PCI (n=3) | **0** ±0 |
| | |
| 60 min post-PCI (n=3) | **0** ±0 |

Comment: Only one plasma sample contained measurable sirolimus level, while all other plasma samples were free from drug. The measured plasma sirolimus concentration was far below the toxic level and much less than used for therapeutic aims.

**Table 14: Balloon surface remnant sirolimus level of GROUP 3 balloon**

| Catheter surface remnant sirolimus amount of GROUP 3 balloon | **Sirolimus Amount [µg]** |
|---|---|
| | |
| Mean±SD | **37.3** ±28.1 |

Comment: Calculating 3 µg sirolimus on the balloon surface (3 mm diameter and 20 mm length), the total amount of the sirolimus on the balloon surface should be 565.2 µg. The balloon surface remnant sirolimus amount was mean 37.3 µg (6.6%). Regarding the amount of the remnant sirolimus on the balloon surface, a second dilation procedure with the same balloon (outside of the 2x30 sec) would not deliver further sufficient amount of sirolimus into the vessel wall.

Considering the amount of tissue, plasma and remnant sirolimus on balloon surface, it seems that the sirolimus drug delivery from drug-coated balloon to arterial tissue is sufficient and in therapeutic range.

## Claims

1. A balloon catheter comprising a coating with an active agent and a water soluble shellac salt.

2. Balloon catheter according to claim 1, wherein the water soluble shellac salt is a shellac ammonium salt.

3. Balloon catheter according to claim 1 or 2, wherein the coating comprises a concentration gradient of the active agent

4. Balloon catheter according to anyone of claim 1 - 3, wherein the concentration gradient of the active agent is in the layer of water soluble shellac salt as a matrix substance.

5. Balloon catheter according to anyone of claim 1 - 4, wherein the active agent is an antirestenotic agent, antiproliferative, immunosuppressive, anti-angiogenic, anti-inflammatory, and/or anti-thrombotic agent.

6. Balloon catheter according to anyone of claim 1 - 5, wherein the active agent is selected from the group consisting of:
abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-AII, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide, cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, β-lapachone, podophyllotoxin, podophyllic acid-2-ethyl hydrazide, rhuGM-CSF, peginterferon α-2b, r-HuG-CSF, macrogol, cytokine antagonist, cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors, paclitaxel, 6-α-hydroxy-paclitaxel, taxoteres, paclitaxel bound to albumin, nap-paclitaxel, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids, bryophyllin A, inotodiol, maquiroside A, ghalakinoside, mansonine, strebloside, hydrocortisone, betamethasone, dexamethasone, non-steroidal substances, fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alphasenecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus, biolimus A9, pimecrolimus, everolimus, zotarolimus, tacrolimus, sirolimus bound to albumin, nap- sirolimus, fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

7. Balloon catheter according to claim 6, wherein the active agent is selected from the group consisting of:
paclitaxel, taxanes, docétaxel, paclitaxel bound to albumin, like nap-paclitaxel, sirolimus, biolimus A9, pimecrolimus, everolimus, zotarolimus, tacrolimus, sirolimus bound to albumin, like nap- sirolimus, fasudil and epothilones.

8. Balloon catheter according to claim 7, wherein the active agent is paclitaxel or sirolimus.

9. Balloon catheter according to anyone of claim 1 - 4, wherein the coating comprises further a water soluble polymer and/or a plasticizer.

10. Balloon catheter according to claim 9, wherein the water soluble polymer is selected from the group comprising cellulose, hydroxypropyl methylcellulose, hydroxypropyl cellulose, carboxymethyl cellulose, polyvinylpyrrolidone, starch, hydroxyl ethyl starch, polyacrylic acid, polyethyleneimine, dextran, agar, carrageenan, alginate, copolymers and/or mixtures of these substances.

11. Method for coating a balloon catheter according to claim 1 comprising the following steps:
IA) providing an uncoated balloon catheter;
and
IIA) providing an aqueous solution of an active agent and a water soluble shellac salt;
or
IIB) providing a solution of the active agent and providing an aqueous solution of a water soluble shellac salt;
and
IIIA) coating the surface of the balloon of the balloon catheter with the aqueous solution of the active agent and the water soluble shellac salt;
or
IIIB) coating the surface of the balloon of the balloon catheter with the solution of
the active agent and subsequently with the aqueous solution of the water soluble shellac salt or coating the surface of the balloon of the balloon catheter with the aqueous solution of the water soluble shellac salt and subsequently with the solution of active agent;
IV) drying the coated balloon,
wherein the aqueous solution of the water soluble shellac salt or the aqueous solution of the active agent and the water soluble shellac salt are prepared using an alkali salt or an ammonium salt of shellac.

12. Method according to claim 11, wherein the solution of the ammonium salt of shellac is a solution of ammonia, ammonium carbonate, or ammonium bicarbonate and shellac.

13. Method according to claim 11 or 12, wherein the active agent is paclitaxel or sirolimus.

14. Method according to any one of claims 11 to 13, wherein the active agent containing solution is applied by means of spray coating, brush coating, vapour deposition or pipetting.

15. Coated balloon catheter which can be obtained by the method according to any one of claims 11 to 14.

## Patentansprüche

1. Ein Ballonkatheter umfassend eine Beschichtung mit einem Wirkstoff und einem wasserlöslichen Schellacksalz.

2. Ballonkatheter gemäß Anspruch 1, wobei das wasserlösliche Schellacksalz ein Schellackammoniumsalz ist.

3. Ballonkatheter gemäß Anspruch 1 oder 2, wobei die Beschichtung einen Konzentrationsgradienten des Wirkstoffs umfasst.

4. Ballonkatheter gemäß einem der Ansprüche 1 - 3, wobei der Konzentrationsgradient des Wirkstoffs in der Schicht des wasserlöslichen Schellacksalzes als eine Matrixsubstanz ist.

5. Ballonkatheter gemäß einem der Ansprüche 1 - 4, wobei der Wirkstoff ein antirestenotisches Agens, antiproliferatives, immunsuppressives, antiangiogenes, anti-inflammatorisches und/oder anti-thrombotisches Agens ist.

6. Ballonkatheter gemäß einem der Ansprüche 1 - 5, wobei der Wirkstoff ausgewählt ist aus der Gruppe bestehend aus:
Abciximab, Acemetacin, Acetylvismion B, Aclarubicin, Ademetionin, Adriamycin, Aescin, Afromoson, Akagerin, Aldesleukin, Amidoron, Aminoglutethimid, Amsacrin, Anakinra, Anastrozol, Anemonin, Anopterin, Antimykotika Antithrombotika, Apocymarin, Argatroban, Aristolactam-AII, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren, Bruceanol A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharanthin, Cerivastatin, CETP-Inhibitoren, Chlorambucil, Chlorochinphosphat, Cicutoxin, Ciprofloxacin, Cisplatin, Cladribin, Clarithromycin, Colchicin, Concanamycin, Coumadin, C-Typ-natriuretisches Peptid, Cudraisoflavon A, Curcumin, Cyclophosphamid, Ciclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Daunamycin, Epirubicin, Erythromycin, Estramustin, Etoposid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegaspargase, Exemestan, Letrozol, Formestan, Mycophenolatmofetil, β-Lapachon, Podophyllotoxin, Podophyllsäure-2-ethylhydrazid, rhuGM-CSF, Peginterferon α-2b, r-HuG-CSF, Macrogol, Cytokinantagonist, Cytokinininhibitoren, COX-2-lnhibitor, Angiopeptin, monoklonale Antikörper, die die Muskelzellproliferation hemmen, bFGF-Antagonisten, Probucol, Prostaglandine, 1-Hydroxy-11-methoxycanthin-6-on, Scopoletin, NO-Donoren, Pentaerythrityltetranitrat und Sydnoimine, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosinkinase-Inhibitoren, Paclitaxel, 6-α-Hydroxypaclitaxel, Taxotere, Paclitaxel gebunden an Albumin, nab-Paclitaxel, Mofebutazon, Lonazolac, Lidocain, Ketoprofen, Mefenaminsäure, Piroxicam, Meloxicam, Penicillamin, Hydroxychlorochin, Natriumaurothiomalat, Oxaceprol, β-Sitosterol, Myrtecain, Polidocanol, Nonivamid, Levomenthol, Ellipticin, Colcemid, Cytochalasin A-E, Indanocin, Nocodazol, Bacitracin, Vitronectin-Rezeptor Antagonisten, Azelastin, Guanidylcyclase-Stimulator, Gewebsinhibitor der Metallproteinase-1 und 2, freie Nukleinsäuren, Nukleinsäuren in Virenüberträger inkorporiert, DNA- Und RNA-Fragmente, Plasminogen-Aktivator Inhibitor-1, Plasminogen-Aktivator Inhibitor-2, Antisense Oligonukleotide, VEGF-Inhibitoren, IGF-1, Wirkstoffe aus der Gruppe der Antibiotika, Cefadroxil, Cefazolin, Cefaclor, Cefoxitin, Tobramycin, Gentamicin, Penicilline, Dicloxacillin, Oxacillin, Sulfonamide, Metronidazol, Enoxaparin, Heparin, Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioprotease-Inhibitoren, Prostacyclin, Vapiprost, Interferon α, β und γ, Histamin-Antagonisten, Serotonin-Blocker, Apoptose-Inhibitoren, Apoptose-Regulatoren, Halofuginon, Nifedipin, Tocopherol, Tranilast, Molsidomin, Teepolyphenole, Epicatechingallat, Epigallocatechingallat, Leflunomid, Etanercept, Sulfasalazin, Tetracyclin, Triamcinolon, Mutamycin, Procainimid, Retinolsäure, Quinidin, Disopyrimid, Flecainid, Propafenon, Sotalol, natürliche und synthetisch hergestellte Steroide, Bryophyllin A, Inotodiol, Maquirosid A, Ghalakinosid, Mansonin, Streblosid, Hydrocortison, Betamethason, Dexamethason, nichtsteroidale Substanzen, Fenoprofen, Ibuprofen, Indomethacin, Naproxen, Phenylbutazon, antivirale Agentien, Acyclovir, Ganciclovir Zidovudin, Clotrimazol, Flucytosin, Griseofulvin, Ketoconazol, Miconazol, Nystatin, Terbinafin, antiprotozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolid, 4,7-Oxycycloanisomelsäure, Baccharinoide B1, B2, B3 and B7, Tubeimosid, Bruceantinosid C, Yadanzioside N und P, Isodeoxyelephantopin, Tomenphantopin A und B, Coronarin A,B C und D, Ursolsäure, Hyptatatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A and B, Dihydronitidin, Nitidinchlorid, 12-β-Hydroxypregnadien-3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Marchantin A, Maytansin, Lycoridicin, Margetin, Pancratistatin, Liriodenin, Oxoushinsunin, Periplocosid A, Deoxypsorospermin, Psychorubin, Ricin A, Sanguinarin, Manwuweizsäure, Methylsorbifolin, Chromone der Spathelia, Stizophyllin, Dihydrousambaraensin, Hydroxyusambarin, Strychnopentamin, Strychnophyllin, Usambarin, Usambarensin, Liriodenin, Daphnoretin, Lariciresinol, Methoxylariciresinol, Syringaresinol, Sirolimus, Biolimus A9, Pimecrolimus, Everolimus, Zotarolimus, Tacrolimus, Sirolimus gebunden an Albumin, nab-Sirolimus, Fasudil, Epothilone, Somatostatin, Roxithromycin, Troleandomycin, Simvastatin, Rosuvastatin, Vinblastin, Vincristin, Vindesin, Teniposid, Vinorelbin, Trofosfamid, Treosulfan, Temozolomid, Thiotepa, Tretinoin, Spiramycin, Umbelliferon, Desacetylvismion A, Vismion A und B, Zeorin.

7. Ballonkatheter gemäß Anspruch 6, wobei der Wirkstoff ausgewählt wird aus der Gruppe bestehend aus:
Paclitaxel, Taxane, Docétaxel, Paclitaxel gebunden an Albumin wie nab-Paclitaxel, Sirolimus, Biolimus A9, Pimecrolimus, Everolimus, Zotarolimus, Tacrolimus, Sirolimus gebunden an Albumin wie nab-Sirolimus, Fasudil und Epothilone.

8. Ballonkatheter gemäß Anspruch 7, wobei der Wirkstoff Paclitaxel oder Sirolimus ist.

9. Ballonkatheter gemäß einem der Ansprüche 1 - 4, wobei die Beschichtung weiter ein wasserlösliches Polymer und/oder einen Weichmacher umfasst.

10. Ballonkatheter gemäß Anspruch 9, wobei das wasserlösliche Polymer ausgewählt ist aus der Gruppe umfassend Cellulose, Hydroxypropylmethylcellulose, Hydroxypropylcellulose, Carboxymethylcellulose, Polyvinylpyrrolidon, Stärke, Hydroxylethylstärke, Polyacrylsäure, Polyethylenimin, Dextran, Agar, Carrageen, Alginat, Copolymere und/oder Mischungen dieser Substanzen.

11. Verfahren zur Beschichtung eines Ballonkatheters gemäß Anspruch 1 umfassend folgende Schritte:
IA) Bereitstellen eines unbeschichteten Ballonkatheters;
und
IIA) Bereitstellen einer wässrigen Lösung eines Wirkstoffs und eines wasserlöslichen Schellacksalzes;
oder
IIB) Bereitstellen einer Lösung eines Wirkstoffs und Bereitstellen einer Lösung eines wasserlöslichen Schellacksalzes;
und
IIIA) Beschichten der Ballonoberfläche des Ballonkatheters mit der wässrigen Lösung des Wirkstoffs und des wasserlöslichen Schellacksalzes;
oder
IIIB) Beschichten der Ballonoberfläche des Ballonkatheters mit der Lösung des Wirkstoffs und anschließend mit der wässrigen Lösung des wasserlöslichen Schellacksalzes oder Beschichten der Ballonoberfläche des Ballonkatheters mit der wässrigen Lösung des wasserlöslichen Schellacksalzes und anschließend mit der Lösung des Wirkstoffs;
IV) Trocknen des beschichteten Ballons,
wobei die wässrige Lösung des wasserlöslichen Schellacksalzes oder die wässrige Lösung des Wirkstoffs und des wasserlöslichen Schellacksalzes mit einem Schellackalkalisalz oder einem Schellackammoniumsalz hergestellt wird.

12. Verfahren gemäß Anspruch 11, wobei die Lösung des Schellackammoniumsalzes eine Lösung aus Ammoniak, Ammoniumcarbonat, oder Ammoniumhydrogencarbonat und Schellack ist.

13. Verfahren gemäß Anspruch 11 oder 12, wobei der Wirkstoff Paclitaxel oder Sirolimus ist.

14. Verfahren gemäß einem der Ansprüche 11 - 13, wobei die Wirkstoff enthaltende Lösung aufgetragen wird mittels Sprühbeschichtung, Streichbeschichtung, Gasphasenabscheidung oder Pipettieren.

15. Beschichteter Ballonkatheter, der nach einem Verfahren gemäß einem der Ansprüche 11 - 14 erhalten werden kann.

## Revendications

1. Un cathéter à ballonnet comprenant un revêtement d'un agent actif et un sel de shellac soluble dans l'eau.

2. Le cathéter à ballonnet selon la revendication 1, dans lequel le sel de shellac soluble dans l'eau est un sel d'ammonium.

3. Le cathéter à ballonnet selon revendication 1 ou 2, dans lequel le revêtement comprend un gradient de concentration de l'agent actif.

4. Le cathéter à ballonnet selon l'une quelconque des revendications 1 à 3, dans lequel le gradient de concentration de l'agent actif se trouve dans la couche de de sel de shellac soluble à l'eau en tant que substance de la matrice.

5. Le cathéter à ballonnet selon l'une quelconque des revendications 1 à 4, dans lequel l'agent actif est un agent anti-resténose, anti-prolifératif, immunosuppresseur, un anti-angiogénique, anti-inflammatoire et / ou anti-thrombotique.

6. Le cathéter à ballonnet selon l'une quelconque des revendications 1 à 5, dans lequel l'agent actif est choisi à partir du groupe constitué par: abciximab, acemétacine, acétylvismione B, aclarubicine, ademétionine, adriamycine, aescin, afromosone, akagerine, aldesleukine, amidorone, aminoglutéthimide, amsacrine, anakinra, anastrozole, anémonin, anopterine, antimycotiques, antithrombotiques, apocymarine, argatroban, aristolactame-All, acide aristolochique, ascomycine, asparaginase, aspirine, atorvastatine, auranofine, azathioprine, azithromycine, baccatin, bafilomycine, basiliximab, bendamustine, benzocaine, berbérine, bétuline, acide bétulinique, bilobol, bisparthenolidine, bléomycine, bombrestatin, acides boswelliques, brucéanol A, B et C, bryophylline A, busulfan, antithrombine, bivalirudine, cadhérines, camptothécine, capécitabine, acide o-carbamoyl-phenoxyacétique, carboplatine, carmustine, célécoxib, cépharanthine, cérivastatine, inhibiteurs de la CETP, chlorambucil, chloroquine phosphate, cicutoxine, ciprofloxacine, cisplatine, cladribine, clarithromycine, colchicine, concanamycine, coumadin, peptide natriurétique de type C (CNP), cudraisoflavone A, curcumine, cyclophosphamide, ciclosporine A, cytarabine, dacarbazine, daclizumab, dactinomycin, dapsone, daunorubicine, diclofenac, 1,11-diméthoxycanthin-6-one, docétaxel, doxorubicine, daunomycine, épirubicine, érythromycine, éstramustine, étoboside, everolimus, filgrastim, fluroblastin, fluvastatin, fludarabine, fludarabine-5'-dihydrogéne phosphate, fluorouracile, folimycine, fosfestrol, gemcitabine, ghalakinoside, ginkgol, acide ginkgolique, glycoside 1a, 4-hydroxyoxycyclo phosphamide, idarubicine, ifosfamide, josamycine, lapachol, lomustine, lovastatin, melphalan, midécamycine, mitoxantrone, nimustine, pitavastatine, pravastatine, procarbazine, mitomycine, méthotrexate, mércaptopurine, thioguanine, oxaliplatin, irinotécan, topotécan, hydroxycarbamide, miltéfosine, pentostatine, pegaspargase, exémestane, létrozole, formestane, mycophénolate mofétil, β-lapachone, podophyllotoxine, acide podophyllique-2-éthylhydrazide, rhuGM-CSF, pegintérferon α-2b, r-HuG-CSF, macrogol, cytokine antagoniste, inhibiteurs des cytokines, inhibiteur de la COX-2, angiopeptine, anticorps monoclonaux qui inhibitent la prolifération des cellules musculaires, antagonistes du bFGF, probucol, prostaglandines, 1-hydroxy-11-méthoxycanthin-6-one, scopolétol, donneurs de NO, pentaérythrityl tetranitrate et sydnoimines, tamoxifène, staurosporine, β-estradiol, α-estradiol, estriol, estrone, éthinyl estradiol, médroxyprogestérone, cypionates d'estradiol, benzoates d'estradiol, tranilast, kamebakaurine et autres terpénoides utilisés dans la thérapie du cancer, verapamil, inhibiteurs de la tyrosine kinase, paclitaxel, 6-α-hydroxy-paclitaxel, taxotères, paclitaxel lié à l'albumine, nappaclitaxel, mofebutazone, lonazolac, lidocaine, kétoprofène, acide méfénamique, piroxicam, méloxicam, pénicillamine, hydroxychloroquine, aurothiomalate de sodium, oxacéprol, β-sitostérol, myrtécaïne, polidocanol, nonivamide, lévomenthol, ellipticine, colcémid, cytochalasines A-E, indanocine, nocodazole, bacitracine, antagonistes du récepteur de la vitronectine, azélastine, stimulateur de la guanidyl cyclase, inhibiteur tissulaire des métalloprotéinases 1 et 2, acides nucléiques libres, acides nucléiques incorporés dans des virus transmetteurs, fragments de l'ADN et l'ARN, inhibiteur-1 de l'activateur du plasminogène, inhibiteur-2 de l'activateur du plasminogène, les oligonucléotides antisens, les inhibiteurs du VEGF, l'IGF-1, agents actifs du groupe des antibiotiques, céfadroxil, céfazolin, céfaclor, céfoxitin, tobramycine, gentamicine, pénicillines, dicloxacilline, oxacilline, sulfonamides, métronidazole, énoxaparin, héparines, hirudine, PPACK, protamine, prourokinase, streptokinase, warfarine, urokinase, vasodilatateurs, dipyramidole, trapidil, nitroprussides, antagonistes du PDGF, triazolopyrimidine, seramin, inhibiteurs de l'ACE, captopril, cilazapril, lisinopril, énalapril, losartan, inhibiteurs de la thioprotéase, prostacycline, vapiprost, interféron α, β et γ, antagonistes d'histamine, bloqueurs de la sérotonine, inhibiteurs de l'apoptose, régulateurs de l'apoptose, halofuginone, nifédipine, tocophérol, tranilast, molsidomine, polyphénols du thé, gallate d'épicatéchine, gallate d'épigallocatéchine, léflunomide, étanercept, sulfasalazine, tétracycline, triamcinolone, mutamycine, procainimide, acide rétinoïque, quinidine, disopyrimide, flécaïnide, propafénone, sotalol, stéroïdes naturels et synthétiques, bryophylline A, inotodiol, maquiroside A, ghalakinoside, mansonine, strebloside, hydrocortisone, bétaméthasone, déxaméthasone, substances non-stéroïdiennes, fénoprofène, ibuprofène, indomèthacine, naproxen, phénylbutazone, agents antiviraux, acyclovir, ganciclovir, zidovudine, clotrimazole, flucytosine, griséofulvine, kétoconazole, miconazole, nystatin, terbinafine, agents antiprotozoaires, chloroquine, méfloquine, quinine, terpénoïdes naturels, hippocaesculine, barringtogénol-C21-angelate, 14-déhydroagrostistachine, agroskerine, agrostistachine, 17-hydroxyagrostistachine, ovatodiolids, acide 4,7-oxycycloanisomelique, baccharinoids B1, B2, B3 et B7, tubeimoside, bruceantinoside C, yadanziosides N et P, isodéoxyelephantopine, tomenphantopine A et B, coronarine A, B, C et D, acide ursolique, acide hyptatique A, iso-iridogermanal, maytenfoliol, effusantine A, excisanine A et B, longikaurine B, sculponeatin C, kamebaunin, leukamenin A et B, 13,18-déhydro-6-alpha-senecioyloxychaparrin, taxamairin A et B, regenilol, triptolide, cymarin, hydroxyanoptérine, protoanémonin, chlorure de cheliburin, sinococuline A et B, dihydronitidine, chlorure de nitidine, 12-β-hydroxypregnadièn-3,20-dione, hélénaline, indicine, indicine-N-oxyde, lasiocarpine, inotodiol, podophyllotoxin, justicidine A et B, larréatin, malloterin, mallotochromanol, isobutyrylmallotochromanol, maquiroside A, marchantine A, maytansine, lycoridicine, margétine, pancratistatin, liriodènine, oxoushinsunine, periplocoside A, déoxypsorospermine, psychorubine, ricin A, sanguinarine, acide du blé manwu, méthylsorbifoline, chromones de spathelia, stizophylline, dihydro usambaraensine, hydroxyusambarine, strychnopentamine, strychnophylline, usambarine, usambarensine, liriodenine, daphnoretine, laricirésinol, méthoxylaricirésinol, syringarésinol, sirolimus, biolimus A9, pimecrolimus, everolimus, zotarolimus, tacrolimus, sirolimus lié à l'albumine, nap-sirolimus, fadusil, épothilones, somatostatine, roxythromycine, troleadomycin, simvastatine, rosuvastatine, vinblastine, vincristine, vindesine, teniposide, vinorelbine, trofosfamide, tréosulfan, trémozolomide, thiotépa, trétinoine, spiramycine, umbellifèrone, desacétylvismione A, vismione A et B, zeorin.

7. Le cathéter à ballonnet selon la revendication 6, dans lequel l'agent actif est choisi à partir du groupe constitué par: paclitaxel, taxanes, docétaxel, paclitaxel lié à l'albumine, comme le nap-paclitael, sirolimus, biolimus A9, pimécrolimus, everolimus, zotarolimus, tacrolimus, sirolimus lié à l'albumine, comme le nap-sirolimus, fadusil et épothilones.

8. Le cathéter à ballonnet selon la revendication 7, dans lequel l'agent actif est paclitaxel ou sirolimus.

9. Le cathéter à ballonnet selon l'une quelconque des revendications 1 à 4, dans lequel le revêtement comprend en outre un polymère soluble dans l'eau et / ou un plastifiant.

10. Le cathéter à ballonnet selon la revendication 9, dans lequel le polymère soluble dans l'eau est choisi à partir du groupe comprenant cellulose, hydroxypropyl méthylcellulose, hydroxypropyl cellulose, carboxyméthyl cellulose, polyvinyle pyrollidones, amidon, hydroxyéthyl amidon, acide polyacrylique, polyéthylèneimine, dextran, agar, carraghénine, alginate, copolymères et / ou mélanges de ces substances.

11. Une méthode pour le revêtement d'un cathéter à ballonnet selon la revendication 1, comprenant les étapes suivantes:
IA) fournir un cathéter à ballonnet non-revêtu;
et
IIA) fournir une solution aqueuse d'un agent actif et d'un sel de shellac soluble dans l'eau;
ou
IIB) fournir une solution de l'agent actif et fournir une solution aquese d'un sel de shellac soluble dans l'eau;
et
IIIA) couvrir la surface du ballonnet du ballonnet à cathéter avec la solution aqueuse de l'agent actif et du sel de shellac soluble dans l'eau;
ou
IIIB) couvrir la surface du ballonnet du ballonnet à cathéter avec la solution de l'agent actif et ensuite avec la solution aqueuse du sel de shellac soluble dans l'eau ou couvrir la surface du ballonnet du ballonnet à cathéter avec la solution aqueuse du sel de shellac soluble dans l'eau et ensuite avec la solution de l'agent actif;
IV) sécher le ballonnet revêtu,
où la solution aqueuse du sel de shellac soluble dans l'eau ou la solution aqueuse de l'agent actif et du sel de shellac soluble dans l'eau sont préparées en utilisant un sel alcalin ou un sel d'ammonium de shellac.

12. La méthode selon la revendication 11, où la solution du sel d'ammonium de shellac est une solution d'ammoniaque, de carbonate d'ammonium ou de bicarbonate d'ammonium et de shellac.

13. La méthode selon la revendication 11 ou 12, dans laquelle l'agent actif est paclitaxel ou sirolimus.

14. La méthode selon l'une quelconque des revendications 11 à 13, dans laquelle la solution contenant l'agent actif est appliquée par pulvérisation, enduction à la brosse, dépôt en phase vapeur ou pipetage.

15. Un cathéter à ballonnet revêtu, qui peut être obtenu par la méthode selon une quelconque des revendications 11 à 14.
